(19) Europäisches Patentamt / European Patent Office / Office européen des brevets

(11) **EP 3 689 291 B1**

(12) **EUROPEAN PATENT SPECIFICATION**

(45) Date of publication and mention
of the grant of the patent:
**31.08.2022 Bulletin 2022/35**

(21) Application number: **18862234.4**

(22) Date of filing: **26.09.2018**

(51) International Patent Classification (IPC):
*A61C 13/087* (2006.01)     *A61C 5/77* (2017.01)
*A61C 8/00* (2006.01)     *A61C 13/00* (2006.01)
*A61C 13/01* (2006.01)     *A61C 13/083* (2006.01)
*A61C 13/08* (2006.01)

(52) Cooperative Patent Classification (CPC):
**A61C 13/0022; A61C 13/081; A61C 13/082**

(86) International application number:
**PCT/JP2018/035790**

(87) International publication number:
**WO 2019/065777 (04.04.2019 Gazette 2019/14)**

(54) **DENTAL MILL BLANK AND METHOD FOR PRODUCING SAME**

DENTALFRÄSROHLING UND VERFAHREN ZU SEINER HERSTELLUNG

CARTON MULTIJET POUR DENTISTERIE, ET PROCÉDÉ DE FABRICATION DE CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.09.2017 JP 2017185421**
**07.12.2017 JP 2017235502**

(43) Date of publication of application:
**05.08.2020 Bulletin 2020/32**

(73) Proprietor: **Kuraray Noritake Dental Inc.**
**Kurashiki-shi**
**Okayama 710-0801 (JP)**

(72) Inventors:
• **INOUE, Masashi**
**Tainai-shi**
**Niigata 959-2653 (JP)**

• **IKEDA, Makoto**
**Tainai-shi**
**Niigata 959-2653 (JP)**
• **ISHINO, Hiroshige**
**Tainai-shi**
**Niigata 959-2653 (JP)**

(74) Representative: **D Young & Co LLP**
**120 Holborn**
**London EC1N 2DY (GB)**

(56) References cited:
WO-A1-2017/154850     WO-A1-2017/154850
JP-A- 2005 514 305     JP-A- 2017 109 036
US-A1- 2003 125 189     US-A1- 2015 238 291
US-A1- 2017 216 000

**Description**

TECHNICAL FIELD

The present invention relates to a dental mill blank, and a method for producing same. Specifically, the present invention relates to a dental mill blank suitable for use in the fabrication of dental prostheses such as inlays, onlays, veneers, crowns, bridges, abutment teeth, dental posts, dentures, denture bases, and implant parts (fixtures, abutments) produced by machining using, for example, the dental CAD/CAM system, and to a method for producing same.

BACKGROUND ART

There has been widespread use of the CAD/CAM system, which is a computer-aided technology for designing and manufacture of inlays, crowns, and other dental prostheses using a milling machine for machining. In the CAD/CAM system, a suitably sized block of a shape such as a cuboid, a cylinder, or a disc is fed and set on a dental milling machine, and milled to obtain a crown- or teeth-shaped restoration. Various block materials have been proposed, including, for example, glass-ceramics, zirconia, titanium, acrylic resins, and composite materials containing polymer resin and inorganic filler.

For example, the dental mill blank producing method described in Patent Literature 1 uses a composite material to enable manufacture of dental prostheses that excel in mechanical strength, abrasion resistance, and surface gloss, and that provide excellent abrasion resistance for opposing teeth. In this method, an inorganic filler molded product prepared by pressing an inorganic filler is contacted with a polymerizable monomer-containing composition, and the polymerizable monomer is polymerized and cured.

WO2017154850 discloses a dental mill blank comprising a mill blank portion (X) and a coating layer (Y) that covers at least all of the circumferential surfaces of the mill blank portion (X), the latter comprising inorganic filler and a cured product of polymerizable monomers (b). The coating layer (Y) comprises a cured product of the polymerizable monomers (b) and is substantially free of inorganic filler.

CITATION LIST

Patent Literature

Patent Literature 1: WO2014/021343A1

SUMMARY OF INVENTION

Technical Problem

The present inventors conducted intensive studies, and found that the method described in Patent Literature 1 potentially involves cracking during polymerization. A dental mill blank with cracks has reduced mechanical strength, and is susceptible to chipping in milling small, delicate parts with the CAD/CAM system, in addition to being not cosmetically appealing. Further improvements are needed in these respects.

The present invention has been made to provide a solution to the foregoing shortcomings of the related art, and it is an object of the present invention to provide a dental mill blank having reduced cracks and that excels in mechanical strength and aesthetic quality.

Solution to Problem

After intensive studies conducted to achieve the foregoing object, the present inventors found that a dental mill blank having reduced cracks and that excels in mechanical strength and aesthetic quality can be obtained with ease when the inorganic filler content in a dental mill blank is confined within a specific range. The present invention was completed after further studies based on this finding.

The present invention is defined in the appended claims.

Advantageous Effects of Invention

The present invention has provided a dental mill blank having reduced cracks and that excels in mechanical strength and aesthetic quality.

DESCRIPTION OF EMBODIMENTS

The present invention is described below in detail.

A dental mill blank of the present invention comprises an inorganic filler and a polymer, and has a porosity of 25 to 35 volume% after 2-hour ashing at 600°C.

With the foregoing configuration, the dental mill blank can have reduced cracks, and excellent mechanical strength and aesthetic quality.

A possible explanation for this effect is as follows, though this is not to be construed as limiting the present invention in any ways. Presumably, cracks in a dental mill blank are caused by stress due to the contraction of the polymerizable monomer during its polymerization, or stress created by local clumping of the polymer produced, among other possible factors. With the post-ashing porosity confined in the specific range, the inorganic fillers appear to be able to tightly pack to such an extent that local clumping of the polymerizable monomer and the polymer becomes less likely to occur, reducing cracks in the dental mill blank, not just in the surface but inside the mill blank. Because the polymer is less likely to clump locally, the mechanical force of engagement between inorganic filler particles increases, which improves ductility, and, in turn, the mechanical strength of the dental mill blank. The inorganic filler also becomes less likely to be removed by external forces such as by toothbrush abrasion, maintaining the high glossiness and high aesthetic quality of the dental mill blank.

Inorganic Filler

The inorganic filler may be known inorganic particles used as fillers for dental composite resins. Examples of such inorganic particles include various types of glasses (for example, glasses containing boron and/or aluminum and various heavy metals in the main component silicon dioxide (e.g., quartz, fused quartz, silica gel) or silicon), alumina, various types of ceramics, diatomaceous earth, kaolin, clay minerals (e.g., montmorillonite), activated earth, synthetic zeolite, mica, silica, calcium fluoride, ytterbium fluoride, calcium phosphate, barium sulfate, zirconium dioxide (zirconia), titanium dioxide (titania), and hydroxyapatite. The inorganic filler also may be organic-inorganic composite particles (organic-inorganic composite filler) obtained as, for example, pulverized particles of a polymer obtained by polymerizing and curing a polymerizable monomer added to inorganic particles such as above. The inorganic filler may be used alone, or two or more thereof may be used in combination.

Transparency and radiopacity are two important properties of crown restoration materials, and, desirably, crown restoration materials should have the same levels of transparency and radiopacity as those of natural teeth. This level of transparency is achievable by matching the refractive indices of the inorganic filler and the polymer as closely as possible. The desired radiopacity can be imparted by using an inorganic filler (e.g., an oxide) containing a heavy metallic element such as zirconium, barium, titanium, lanthanum, or strontium. Typically, such an inorganic filler containing a heavy metallic element has a high refractive index in a range of 1.5 to 1.6. In the present invention, in the case where, for example, a (meth)acrylate polymerizable monomer is used as a polymerizable monomer that forms a polymer, a polymer of the (meth)acrylate polymerizable monomer has a refractive index typically in a range of 1.5 to 1.6, and a small refractive index difference can be achieved even when it is combined with a high-refractive-index inorganic filler having radiopacity. This makes it possible to produce a dental mill blank having improved transparency.

Examples of such high-refractive-index inorganic fillers capable of imparting radiopacity include barium borosilicate glass (for example, E3000 manufactured by Esstech, and 8235, GM27884, and GM39923 manufactured by Schott), strontium boroaluminosilicate glass (for example, E4000 manufactured by Esstech, and G018-093 and GM32087 manufactured by Schott), lanthanum glass (for example, GM31684 manufactured by Schott), fluoroaluminosilicate glass (for example, G018-091 and G018-117 manufactured by Schott), zirconia-containing glass (for example, G018-310 and G018-159 manufactured by Schott), strontium-containing glass (for example, G018-163, G018-093, and GM32087 manufactured by Schott), zinc oxide-containing glass (for example, G0 18-161 manufactured by Schott), and calcium-containing glass (for example, G018-309 manufactured by Schott).

The shape of the inorganic filler is not particularly limited, and the inorganic filler may have a variety of shapes, including, for example, fragments, plates, scales, fibers (e.g., short fibers, long fibers), styluses, whiskers, and spheres. The inorganic filler may have a form of an aggregate of primary particles of a shape such as above, or may be a combination of different shapes. The inorganic filler may be one that has been processed into a shape such as above by being subjected to some kind of process (for example, pulverization).

According to the invention, in view of advantages such as more efficiently obtaining a dental mill blank of the present invention,the inorganic filler comprises an inorganic filler (A) and an inorganic filler (B), and satisfy the following formulae (I) and (II),

$$0.12 \ \le \ a \ \le \ 0.70 \qquad\qquad \text{(I)}$$

$$3 \leq b/a \qquad \text{(II)},$$

where a is the average primary particle diameter of the inorganic filler (A) in micrometers, and b is the average primary particle diameter of the inorganic filler (B) in micrometers.

The average primary particle diameter (a) of the inorganic filler (A) falls in a range of 0.12 to 0.70 μm. With the inorganic filler (A) having an average primary particle diameter in this range, a dental mill blank of the present invention can be obtained more efficiently, and can have improved mechanical strength and aesthetic quality. In view of this, the average primary particle diameter (a) of inorganic filler (A) is preferably 0.15 μm or more, and is preferably 0.50 μm or less.

Preferably, the average primary particle diameter (b) of inorganic filler (B) satisfies the following formula (III).

$$0.8 \leq b \leq 10 \qquad \text{(III)}$$

Specifically, the average primary particle diameter (b) of inorganic filler (B) falls in a range of preferably 0.8 to 10 μm. With the inorganic filler (B) having an average primary particle diameter in this range, a dental mill blank of the present invention can be obtained more efficiently, and can have improved mechanical strength and aesthetic quality. In view of this, the average primary particle diameter (b) of inorganic filler (B) is preferably 1.0 μm or more, and is preferably 5.0 μm or less.

The average primary particle diameter of the inorganic filler can be determined by using a laser diffraction scattering method. Specifically, for example, the average primary particle diameter of the inorganic filler can be measured with a laser diffraction particle size distribution analyzer (SALD-2100, manufactured by Shimadzu Corporation), using a 0.2% aqueous solution of sodium hexametaphosphate as a dispersion medium.

The average primary particle diameter (b) of inorganic filler (B) is greater than the average primary particle diameter (a) of inorganic filler (A) by a factor of 3 or more. A dental mill blank of the present invention can be obtained more efficiently with the ratio b/a confined in the range of the formula (II). In view of this, the ratio b/a is preferably 5 or more, more preferably 7 or more. The upper limit of b/a is not particularly limited, and may be, for example, 70 or less, 40 or less, 25 or less, or 15 or less.

In the dental mill blank, it is preferable that the inorganic filler (A) and the inorganic filler (B) have a volume-to-volume content ratio (A)/(B) of 5/95 to 50/50. For advantages such as enhancing the effects of the present invention, the volume-to-volume content ratio (A)/(B) of inorganic filler (A) and inorganic filler (B) is more preferably 10/90 or more, even more preferably 15/85 or more, particularly preferably 20/80 or more, and is more preferably 40/60 or less, even more preferably 35/65 or less, particularly preferably 30/70 or less. The volume contents of inorganic filler (A) and inorganic filler (B) can be determined from values obtained by dividing the mass of each filler by the density of the material constituting the corresponding inorganic filler. When the inorganic filler is one obtained after a surface treatment (described later), the proportion of each filler may be calculated using the content of inorganic filler (A) or (B) before surface treatment. When the inorganic filler is forming an inorganic filler complex having a binder on its surface (described later), the proportion of each filler may be calculated using the content of inorganic filler (A) or (B) excluding the binder. The volume contents of inorganic filler (A) and inorganic filler (B) can be determined by, for example, imaging a cross section of the dental mill blank with an electron scanning microscope.

A dental mill blank of the present invention may comprise an inorganic filler other than the inorganic fillers (A) and (B). For advantages such as enhancing the effects of the present invention, the combined proportion of inorganic fillers (A) and (B) in all inorganic fillers contained in a dental mill blank of the present invention is preferably 50 mass% or more, more preferably 80 mass% or more, even more preferably 90 mass% or more, and may be 95 mass% or more, 98 mass% or more, or 100 mass%.

Surface Treatment

The inorganic filler is preferably one subjected to a surface treatment in advance. The dental mill blank produced can have improved mechanical strength by using an inorganic filler that has been subjected to a surface treatment. Another advantage is that the affinity between a polymerizable monomer-containing composition (described later) and the surface of the inorganic filler improves, and the polymerizable monomer-containing composition is able to more easily penetrate into spaces between inorganic filler particles upon being contacted with an inorganic filler molded product prepared by pressing the inorganic filler.

When using two or more inorganic fillers, only one of the inorganic fillers may be subjected to a surface treatment, or all of the inorganic fillers may be subjected to a surface treatment. In the case of the latter, the inorganic fillers may prepared by being individually subjected to a surface treatment, or by performing a surface treatment for a mixture of inorganic fillers.

The surface treatment agent used for surface treatment may be a known surface treatment agent. Examples of such surface treatment agents include: organometallic compounds such as organosilicon compounds, organotitanium compounds, organozirconium compounds, and organoaluminum compounds; and organic compounds having at least one acidic group such as a phosphoric acid group, a pyrophosphoric acid group, a thiophosphoric acid group, a phosphonic acid group, a sulfonic acid group, or a carboxylic acid group (acidic group-containing organic compounds). The surface treatment agent may be used alone, or two or more thereof may be used in combination. When using two or more surface treatment agents in combination, the surface treatment layer may be a layer formed by a mixture of two or more surface treatment agents, or may be a laminate structure of multiple surface treatment layers. The surface treatment method is not particularly limited, and a known method may be used.

Examples of the organosilicon compounds include compounds represented by, for example, $R^1{}_nSiX_{4-n}$ (wherein $R^1$ is a substituted or unsubstituted hydrocarbon group having 1 to 12 carbon atoms, X represents an alkoxy group having 1 to 4 carbon atoms, an acetoxy group, a hydroxyl group, a halogen atom, or a hydrogen atom, and n is an integer of 0 to 3, in which $R^1$ may be the same or different when a plurality of $R^1$ exists, and X may be the same or different when a plurality of X exists).

Specific examples of the organosilicon compounds include methyltrimethoxysilane, dimethyldimethoxysilane, phenyltrimethoxysilane, diphenyldimethoxysilane, methyltriethoxysilane, dimethyldiethoxysilane, phenyltriethoxysilane, diphenyldiethoxysilane, isobutyltrimethoxysilane, vinyltrimethoxysilane, vinyltriethoxysilane, vinyl-tris(β-methoxyethoxy)silane, 3,3,3-trifluoropropyltrimethoxysilane, methyl-3,3,3-trifluoropropyldimethoxysilane, β-(3,4-epoxycyclohexyl)ethyltrimethoxysilane, γ-glycidoxypropyltrimethoxysilane, γ-glycidoxypropylmethyldiethoxysilane, γ-glycidoxypropyltriethoxysilane, γ-methacryloyloxypropylmethyldimethoxysilane, γ-methacryloyloxypropylmethyl diethoxysilane, N-(β-aminoethyl)y-aminopropylmethyldimethoxysilane, N-(β-aminoethyl)y-aminopropyltrimethoxysilane, N-(β-aminoethyl)y-aminopropyltriethoxysilane, γ-aminopropyltrimethoxysilane, γ-aminopropyltriethoxysilane, N-phenyl-γ-aminopropyltrimethoxysilane, γ-mercaptopropyltrimethoxysilane, trimethylsilanol, methyltrichlorosilane, methyldichlorosilane, dimethyldichlorosilane, trimethylchlorosilane, phenyltrichlorosilane, diphenyldichlorosilane, vinyltrichlorosilane, trimethylbromosilane, diethylsilane, vinyltriacetoxysilane, ω-(meth)acryloyloxyalkyltrimethoxysilane (having 3 to 12 carbon atoms between the (meth)acryloyloxy group and the silicon atom; e.g., γ-methacryloyloxypropyltrimethoxysilane), and ω-(meth)acryloyloxyalkyltriethoxysilane (having 3 to 12 carbon atoms between the (meth)acryloyloxy group and the silicon atom; e.g., γ-methacryloyloxypropyltriethoxysilane). As used herein, "(meth)acryloyl" is meant to be inclusive of both "methacryloyl" and "acryloyl".

For advantages such as strengthening the chemical bonding between the inorganic filler and the polymerizable monomer and further improving the mechanical strength of the dental mill blank, the organosilicon compounds are preferably those having a functional group that is copolymerizable with the polymerizable monomer, more preferably ω-(meth)acryloyloxyalkyltrimethoxysflane (having 3 to 12 carbon atoms between the (meth)acryloyloxy group and the silicon atom), ω-(meth)acryloyloxyalkyltriethoxysilane (having 3 to 12 carbon atoms between the (meth)acryloyloxy group and the silicon atom), vinyltrimethoxysilane, vinyltriethoxysilane, vinyltriacetoxysilane, and γ-glycidoxypropyltrimethoxysilane.

Examples of the organotitanium compounds include tetramethyl titanate, tetraisopropyl titanate, tetra-n-butyl titanate, a butyl titanate dimer, and tetra(2-ethylhexyl)titanate.

Examples of the organozirconium compounds include zirconium isopropoxide, zirconium n-butoxide, zirconium acetylacetonate, and zirconium acetate.

Examples of the organoaluminum compounds include aluminum acetylacetonate, and an aluminum-organic acid salt chelate compound.

Examples of the organic compounds having a phosphoric acid group(s) include 2-ethylhexyl acid phosphate, stearyl acid phosphate, 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethylphenyl hydrogen phosphate, 2-(meth)acryloyloxyethyl-2-bromoethyl hydrogen phosphate, bis[2-(meth)acryloyloxy-(1-hydroxymethyl)ethyl]hydrogen phosphate, and acid chlorides, alkali metal salts, ammonium salts thereof.

Aside from the foregoing organic compounds having an acidic group(s) such as a pyrophosphoric acid group, a thiophosphoric acid group, a phosphonic acid group, a sulfonic acid group, and a carboxylic acid group, the acidic group-containing organic compounds mentioned in, for example, WO2012/042911 may be used.

The amount of surface treatment agent is not particularly limited, and is preferably, for example, 0.1 to 50 parts by mass relative to 100 parts by mass of the inorganic filler before surface treatment.

Binder

The inorganic filler may be one forming an inorganic filler complex having a binder on its surface. An inorganic filler complex having a binder on its surface can be obtained by mixing the inorganic filler with a binder. By using an inorganic filler complex having a binder on its surface, the inorganic filler (inorganic filler complex) can be pressed with improved formability, and cracking and chipping in the resulting inorganic filler molded product can be more effectively reduced. Another advantage is that the affinity between a polymerizable monomer-containing composition (described later) and the surface of the inorganic filler improves when contacting the inorganic filler molded product and the polymerizable monomer-containing composition with each other for penetration of the polymerizable monomer-containing composition into spaces between inorganic filler particles. This improves the mechanical strength of the dental mill blank obtained. When the inorganic filler is containing inorganic filler (A) and inorganic filler (B), only one of the inorganic filler (A) and the inorganic filler (B) may be forming an inorganic filler complex having a binder on its surface. It is, however, preferable that an inorganic filler complex having a binder on its surface be formed by both inorganic filler (A) and inorganic filler (B).

The binder is not limited to a particular kind of binder, and may be, for example, a polymerizable monomer (e.g., a radical polymerizable monomer, a cationic polymerizable monomer), a polymer of a polymerizable monomer, a wax, or a plasticizer. The binder may be used alone, or two or more thereof may be used in combination. For advantages such as further improving the mechanical strength of a dental mill blank produced by thermal polymerization and curing of a polymerizable monomer such as by a method that includes the step of contacting the inorganic filler molded product and the polymerizable monomer-containing composition with each other and polymerizing and curing the polymerizable monomer, preferred as the binder contained in the inorganic filler complex before polymerization and curing (for example, at the time of pressing) is at least one selected from the group consisting of a polymerizable monomer, a wax, and a plasticizer, more preferably at least one selected from the group consisting of a polymerizable monomer and a plasticizer, even more preferably a polymerizable monomer. The binder contained in the inorganic filler complex after polymerization and curing (for example, the binder contained in the dental mill blank produced) is preferably at least one selected from the group consisting of a polymer of a polymerizable monomer, a wax, and a plasticizer, more preferably at least one selected from the group consisting of a polymer of a polymerizable monomer, and a plasticizer, even more preferably a polymer of a polymerizable monomer.

Specific examples of the polymerizable monomer used as the binder include polymerizable monomers mentioned below as polymerizable monomers that form the polymer contained in the dental mill blank. Preferred are radical polymerizable monomers. The polymerizable monomer used as the binder is preferably at least one of polymerizable monomers that form the polymer contained in the product dental mill blank (typically, a polymerizable monomer contained in the polymerizable monomer-containing composition described below). Likewise, specific examples of the polymer used as the binder include polymers obtained through polymerization of polymerizable monomers mentioned below as polymerizable monomers that form the polymer contained in the dental mill blank. Preferred are polymers obtained through polymerization of radical polymerizable monomers. The polymer used as the binder is preferably a polymer obtained through polymerization of at least one of polymerizable monomers that form the polymer contained in the product dental mill blank (typically, a polymerizable monomer contained in the polymerizable monomer-containing composition described below).

The polymerizable monomer is preferably a bifunctional (meth)acrylate polymerizable monomer, more preferably 2,2-bis[4-[3-acryloyloxy-2-hydroxypropoxy]phenyl]propane, 2,2-bis[4-[3-methacryloyloxy-2-hydroxypropoxy]phenyl]propane (commonly known as Bis-GMA), 2,2-bis[4-(meth)acryloyloxyethoxyphenyl]propane, 2,2-bis[4- (meth) acryloyloxypolyethoxyphenyl]prop ane, or [2,2,4-trimethylhexamethylenebis(2-carbamoyloxyethyl)]dimethacrylate (commonly known as UDMA), though it depends on factors such as the type of the polymerizable monomer that forms the polymer contained in the dental mill blank.

Examples of the wax include paraffin wax, polyethylene wax, polyolefin wax, and liquid paraffin wax.

Examples of the plasticizer include phthalic acid esters such as dimethyl phthalate, diethyl phthalate, dibutyl phthalate, di(2-ethylhexyl) phthalate, di-n-octyl phthalate, diisononyl phthalate, dinonyl phthalate, diisodecyl phthalate, and butyl benzyl phthalate; adipic acid esters such as dioctyl adipate, diisononyl adipate, di-n-hexyl adipate, and di-n-decyl adipate; and polyethylene glycol (PEG).

The amount of binder is not particularly limited. However, the amount of binder, in terms of a content in the inorganic filler complex, is preferably 0.1 mass% or more, more preferably 0.3 mass% or more, even more preferably 0.5 mass% or more, and is preferably 10 mass% or less, more preferably 5 mass% or less, even more preferably 3 mass% or less. With these lower limits of binder content, the binder is able to more effectively exhibit its effect. With the foregoing upper limits of binder content, the fluidity improves, and a dental mill blank having excellent mechanical strength with reduced cracks and chipping can be produced with improved yield.

A dental mill blank of the present invention requires a porosity of 25 volume% or more after 2-hour ashing at 600°C, and a porosity of 35 volume% or less after 2-hour ashing at 600°C. The porosity is preferably 28 volume% or more, and is preferably 33 volume% or less. In this way, the dental mill blank can have excellent mechanical strength and aesthetic quality with reduced surface and internal cracks. The ashing can be achieved by heating the dental mill blank at 600°C for 2 hours in the presence of oxygen, such as in an air atmosphere. Ashing of an inorganic filler- and polymer-containing dental mill blank at 600°C for 2 hours typically burns the organic components, including the polymer, and creates voids in the burned empty spaces. This means that the value obtained by subtracting the porosity from 100 volume% roughly corresponds to the content (volume%) of the inorganic filler in the dental mill blank. When the inorganic filler is one that has been subjected to a surface treatment or when using an inorganic filler complex having a binder on its surface, the components derived from the surface treatment agent and the binder are typically burned as organic components upon ashing the dental mill blank under the foregoing conditions.

The porosity can be determined as follows. Specifically, the porosity can be calculated using the following formula (IV):

$$\text{Porosity (volume\%)} = \{1 - (m1/m0)\} \times 100 \qquad \text{(IV)},$$

where m1 (g/cm$^3$) is the density of the ashed product after 2-hour ashing of a dental mill blank at 600°C, and m0 (g/cm$^3$) is the true density of the ashed product.

The true density m0 of the ashed product can be determined as follows. Specifically, the true density m0 (g/cm$^3$) can be calculated using the following formula (V):

$$m0 \ (g/cm^3) = \{(W1 - W0)/(W1 - W0 - W2 + W3)\} \times D \qquad \text{(V)},$$

where W0 (g) is the mass of a weighing container, W1 (g) is the mass of the ashed product prepared into a powder form and charged into the container plus the mass of the container, W2 (g) is the mass of a standard liquid (for example, isooctane) put in the container plus the combined mass of the container and the powder after the standard liquid is brought to a certain temperature T (°C) to completely replace air in the voids present in the powder (the volume of the contents of the container is V2 (cm$^3$)), W3 (g) is the mass of a standard liquid solely charged into the container in volume V2 (cm$^3$) plus the mass of the container after the standard liquid is brought to temperature T (°C), and D (g/cm$^3$) is the density of the standard liquid at temperature T (°C).

Polymer

The constituent polymer of a dental mill blank of the present invention is not particularly limited, and may be a polymer of a polymerizable monomer, preferably a polymer resulting from polymerization and curing of a polymerizable monomer contained in the polymerizable monomer-containing composition.

Polymerizable Monomer

The polymerizable monomer that forms the polymer (the polymerizable monomer that forms the structural unit contained in the polymer) may be a known polymerizable monomer used for applications such as dental composite resins. Typically, preferred for use are radical polymerizable monomers. Specific examples of the radical polymerizable monomers include esters of carboxylic acids such as α-cyanoacrylic acid, (meth)acrylic acid, α-halogenated acrylic acid, crotonic acid, cinnamic acid, sorbic acid, maleic acid, and itaconic acid; (meth)acrylamide polymerizable monomers such as (meth)acrylamide, and derivatives of (meth)acrylamide; vinyl esters; vinyl ethers; mono-N-vinyl derivatives; and styrene derivatives. Preferred are one or more polymerizable monomers selected from the group consisting of a (meth)acrylate polymerizable monomer and a (meth)acrylamide polymerizable monomer, more preferably at least one selected from the group consisting of a (meth)acrylate and a (meth)acrylamide derivative, even more preferably (meth)acrylates. As used herein, "(meth)acryl" is meant to be inclusive of both methacryl and acryl. As used herein, "(meth)acrylate" is meant to be inclusive of both methacrylate and acrylate. Examples of the (meth)acrylate and (meth)acrylamide polymerizable monomers are as follows.

(i) Monofunctional (meth)acrylate and (meth)acrylamide polymerizable monomers

Examples include methyl(meth)acrylate, isobutyl(meth)acrylate, benzyl(meth)acrylate, lauryl(meth)acrylate, 2-(N,N-dimethylamino)ethyl(meth)acrylate, 2,3-dibromopropyl(meth)acrylate, 2-hydroxyethyl(meth)acrylate, 6-hydroxyhexyl(meth)acrylate, 10-hydroxydecyl(meth)acrylate, propylene glycol mono(meth)acrylate, glycerin mono(meth)acrylate, erythritol mono (meth)acrylate, (meth)acrylamide, N-methylol (meth)acrylamide, N-hydroxyethyl (meth)acrylamide, N-dihydroxyethyl (meth)acrylamide, (meth)acryloyloxy dodecylpyridinium bromide, (meth)acryloyloxy dodecylpyridinium chloride, (meth)acryloyloxy hexadecylpyridinium chloride, (meth)acryloyloxy decylammonium chloride, and 10-mercaptodecyl (meth)acrylate.

(ii) Bifunctional (meth)acrylate polymerizable monomers

Examples include ethylene glycol di(meth)acrylate, triethylene glycol di(meth)acrylate, propylene glycol di(meth)acrylate, neopentyl glycol di(meth)acrylate, 1,6-hexanediol di(meth)acrylate, 1,10-decanediol di(meth)acrylate, 2,2-bis[4-[3-acryloyloxy-2-hydroxypropoxy]phenyl]propane, 2,2-bis[4-[3-methacryloyloxy-2-hydroxypropoxy]phenyl]propane (commonly known as Bis-GMA), 2,2-bis[4-(meth)acryloyloxyethoxyphenyl]propane, 2,2-bis[4-(meth)acryloyloxypolyethoxyphenyl]propane (for example, the average number of moles of ethoxy group added is 2.6), I,2-bis[3-(meth)acryloyloxy-2-hydroxypropoxy]ethane, pentaerythritol di(meth)acrylate, [2,2,4-trimethylhexamethylenebis(2-carbamoyloxyethyl)] dimethacrylate (commonly known as UDMA), and 2,2,3,3,4,4-hexafluoro-1,5-pentyl di(meth)acrylate.

(iii) Tri- and higher-functional (meth)acrylate polymerizable monomers

Examples include trimethylolpropane tri(meth)acrylate, trimethylolethane tri(meth)acrylate, tetramethylolmethane tri(meth)acrylate, pentaerythritoltetra(meth)acrylate, dipentaerythritol hexa(meth)acrylate, N,N'-(2,2,4-trimethylhexamethylene)bis[2-(aminocarboxy)propane-1,3-diol]tetra(met h)acrylate, and 1,7-diacryloyloxy-2,2,6,6-tetra(meth)acryloyloxymethyl-4-oxyheptane.

Aside from the radical polymerizable monomers, cationic polymerizable monomers such as oxirane compounds and oxetane compounds may be used as polymerizable monomers.

The polymerizable monomer may be used alone, or two or more thereof may be used in combination. Preferably, the polymerizable monomer is liquid form. However, the polymerizable monomer is not necessarily required to be liquid form at ordinary temperature, and it is also possible to use a solid polymerizable monomer, or a dissolved mixture with other liquid polymerizable monomers.

The polymerizable monomer has a viscosity (25°C) of preferably 10 Pa·s or less, more preferably 5 Pa·s or less, even more preferably 2 Pa·s or less. When two or more polymerizable monomers are used as a mixture, or when the polymerizable monomer(s) are used by being diluted with a solvent, it is not required to confine the viscosities of individual polymerizable monomers in these ranges. It is, however, preferable that the viscosity of the polymerizable monomer(s) fall in the foregoing ranges in a ready-to-use form (a mixed or diluted form).

Polymerization Initiator

For ease of polymerization, a polymerization initiator may be used in obtaining a polymer through polymerization of the polymerizable monomer. Particularly, when the polymerizable monomer in the polymerizable monomer-containing composition is polymerized and cured to obtain a polymer, it is preferable that the polymerizable monomer-containing composition additionally contain a polymerization initiator. The polymerization initiator may be selected from polymerization initiators commonly used in industry, preferably from those used in dentistry. For example, the polymerization initiator may be at least one selected from the group consisting of a thermal polymerization initiator, a photopolymerization initiator, and a chemical polymerization initiator.

(i) Thermal Polymerization Initiator

Examples of the thermal polymerization initiator include organic peroxides and azo compounds.

Examples of the organic peroxides include ketone peroxides, hydroperoxides, diacyl peroxides, dialkyl peroxides, peroxy ketals, peroxy esters, and peroxydicarbonates.

Examples of the ketone peroxides include methyl ethyl ketone peroxide, methyl isobutyl ketone peroxide, methylcyclohexanone peroxide, and cyclohexanone peroxide.

Examples of the hydroperoxides include

2,5-dimethylhexane-2,5-dihydroperoxide, diisopropylbenzene hydroperoxide, cumene hydroperoxide, t-butyl hydroperoxide, and 1,1,3,3-tetramethylbutyl hydroperoxide.

Examples of the diacyl peroxides include acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

Examples of the dialkyl peroxides include di-t-butyl peroxide, dicumyl peroxide, t-butylcumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne.

Examples of the peroxyketals include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and n-butyl 4,4-bis(t-butylperoxy)valerate.

Examples of the peroxy esters include α-cumyl peroxyneodecanoate, t-butyl peroxyneodecanoate, t-butyl peroxypivalate, 2,2,4-trimethylpentyl peroxy-2-ethyl hexanoate, t-amyl peroxy-2-ethyl hexanoate, t-butyl peroxy-2-ethyl hexanoate, di-t-butyl peroxyisophthalate, di-t-butyl peroxyhexahydroterephthalate, t-butyl peroxy-3,3,5-trimethyl hexanoate, t-butyl peroxyacetate, t-butyl peroxybenzoate, and t-butyl peroxymaleate.

Examples of the peroxydicarbonates include di-3-methoxyperoxydicarbonate, di-2-ethylhexyl peroxydicarbonate, bis(4-t-butylcyclohexyl)peroxydicarbonate, diisopropyl peroxydicarbonate, di-n-propyl peroxydicarbonate, di-2-ethoxyethyl peroxydicarbonate, and diallyl peroxydicarbonate.

Of these organic peroxides, preferred for an overall balance of safety, storage stability, and radical generating potential are diacyl peroxides, particularly benzoyl peroxide.

Examples of the azo compounds include 2,2'-azobis(isobutyronitrile), 2,2'-azobis(2,4-dimethylvaleronitrile), 4,4'-azobis(4-cyanovaleric acid), 1,1'-azobis(1-cyclohexanecarbonitrile), dimethyl-2,2'-azobis(isobutyrate), and 2,2'-azobis(2-amidinopropane)dihydrochloride.

(ii) Photopolymerization Initiator

Suitable as the photopolymerization initiator are those widely used for curable compositions in dentistry, for example, such as (bis)acylphosphine oxides, α-diketones, and coumarins.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyl di(2,6-dimethylphenyl)phosphonate, and salts thereof.

Examples of bisacylphosphine oxides in the (bis)acylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, bis(2,3,6-trimethylbenzoyl)-2,4,4-trimethylpentylphosphine oxide, and salts thereof.

Preferred as the (bis)acylphosphine oxides are sodium salts of 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and 2,4,6-trimethylbenzoylphenylphosphine oxide.

Examples of the α-diketones include diacetyl, benzyl, camphorquinone, 2,3-pentadione, 2,3-octadione, 9,10-phenanthrenequinone, 4,4'-oxybenzyl, and acenaphthenequinone. Preferred is camphorquinone.

Examples of the coumarins include 3,3'-carbonylbis(7-diethylaminocoumarin), 3-(4-methoxybenzoyl)coumarin, 3-thienoyl coumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[fl]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazoyl)-7-(diethylamino)coumarin, 3-(2-benzothiazoyl)-7-(dibutylamino)coumarin, 3-(2-benzoimidazolyl)-7-(diethylamino)coumarin, 3-(2-benzothiazoyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetram ethyl-lH,5H,11H-[1]benzopyrrano[6,7,8-ij]quinolizin11-one, and 10-(2-benzothiazoyl)-2,3,6,7-tetrahydro-1,1,7,7-tetramethyl-1H,5H,11H-[1]benzopyrr ano[6,7,8-ij]quinolizin-11-one.

Preferred among these coumarin compounds are 3,3'-carbonylbis(7-diethylaminocoumarin) and 3,3'-carbonylbis(7-dibutylaminocoumarin).

(iii) Chemical Polymerization Initiator

Examples of the chemical polymerization initiator include redox polymerization initiators. The preferred redox polymerization initiators are, for example, organic peroxide-amine polymerization initiators; and organic peroxide-amine-sulfinic acid (or a salt thereof) polymerization initiators. When using a redox polymerization initiator, it is preferable to prepare the oxidizing agent and the reducing agent as separate packages, and mix the two immediately before use.

Examples of the oxidizing agent of redox polymerization initiators include organic peroxides. The organic peroxides may be known organic peroxides. Specifically, the organic peroxides exemplified above in conjunction with the thermal polymerization initiator may be used. For an overall balance of safety, storage stability, and radical generating potential, the organic peroxides are preferably diacyl peroxides, particularly benzoyl peroxide.

The reducing agent of redox polymerization initiators is typically a tertiary aromatic amine having no electron withdrawing group on the aromatic ring. Examples of tertiary aromatic amines having no electron withdrawing group on the aromatic ring include N,N-dimethylaniline, N,N-dimethyl-p-toluidine, N,N-dimethyl-m-toluidine, N,N-diethyl-p-toluidine, N,N-dimethyl-3,5-dimethylaniline,N,N-dimethyl-3,4-dimethylaniline, N,N-dimethyl-4-ethylaniline, N,N-dimethyl-4-isopropylaniline, N,N-dimethyl-4-t-butylanihne,N,N-dimethyl-3,5-di-t-butylaniline, N,N-bis(2-hydroxyethyl)-3,5-dimethylaniline,N,N-bis(2 -hydroxyethyl)-p -toluidine, N,N-bis(2-hydroxyethyl)-3,4-dimethylaniline, N,N-bis(2-hydroxyethyl)-4-ethylanihne,N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, and N,N-bis(2-hydroxyethyl)-3,5-di-t-butylaniline.

The polymerization initiator may be used alone, or two or more thereof may be used in combination. For example, the polymerization initiator may be a combination of a thermal polymerization initiator and a photopolymerization initiator. In this case, it is preferable to combine a diacyl peroxide and a (bis)acylphosphine oxide.

The amount of polymerization initiator is not particularly limited. However, in view of considerations such as ease of polymerization, the polymerization initiator is preferably 0.001 parts by mass or more, more preferably 0.05 parts by mass or more, even more preferably 0.1 parts by mass or more relative to 100 parts by mass of the polymerizable monomer. With these lower limits of polymerization initiator content, polymerization sufficiently takes place even when the polymerization initiator itself is not highly polymerizable, and the resulting dental mill blank, and, in turn, the dental prosthesis produced therefrom, can have improved strength. The polymerization initiator is preferably 30 parts by mass or less, more preferably 20 parts by mass or less relative to 100 parts by mass of the polymerizable monomer. With the foregoing upper limits of polymerization initiator content, it is possible to inhibit precipitation of polymerization initiator.

Polymerization Accelerator

When using a polymerization initiator, the polymerization initiator may be used with a polymerization accelerator. Particularly, when the polymerizable monomer in the polymerizable monomer-containing composition is polymerized and cured to obtain a polymer, the polymerizable monomer-containing composition may also contain a polymerization accelerator, in addition to the polymerization initiator. By using a polymerization accelerator with the polymerization initiator, polymerization can take place more quickly and more efficiently. The polymerization accelerator may be selected from polymerization accelerators commonly used in industry, preferably from those used in dentistry. The polymerization accelerator may be used alone, or two or more thereof may be used in combination.

Examples of polymerization accelerators preferred for use with the photopolymerization initiator include tertiary amines, aldehydes, thiols, and sulfinic acid and salts thereof.

Examples of the tertiary amines include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline,N,N-dimethyl-3,5-di-t-butylaniline, N,N-bis(2-hydroxyethyl)-3,5-dimethylaniline,N,N-bis(2 -hydroxyethyl)-p -toluidine, N,N-bis(2-hydroxyethyl)-3,4-dimethylaniline, N,N-bis(2-hydroxyethyl)-4-ethylaniline,N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylanihne, n-butoxyethyl 4-(N,N-dimethylamino)benzoate, (2-methacryloyloxy)ethyl 4-(N,N-dimethylamino)benzoate, ethyl 4-(N,N-dimethylamino)benzoate, butyl 4-(N,N-dimethylamino)benzoate, N-methyldiethanolamine, 4-(N,N-dimethylamino)benzophenone, trimethylamine, triethylamine, N-methyldiethanolamine, N-ethyldiethanolamine, N-n-butyldiethanolamine, N-lauryldiethanolamine, triethanolamine, 2 -(dimethylamino)ethylmethacrylate, N-methyldiethanolamine dimethacrylate, N-ethyldiethanolamine dimethacrylate, triethanolamine monomethacrylate, triethanolamine dimethacrylate, and triethanolamine trimethacrylate.

Examples of the aldehydes include dimethylaminobenzaldehyde and terephthalaldehyde.

Examples of the thiols include 2-mercaptobenzooxazole, decanethiol, 3-mercaptopropyltrimethoxysilane, and thiobenzoic acid.

Examples of the sulfinic acid and salts thereof include benzenesulfinic acid, sodium benzenesulfinate, potassium benzenesulfinate, calcium benzenesulfinate, lithium benzenesulfinate, p-toluenesulfinic acid, sodium p-toluenesulfinate, potassium p-toluenesulfinate, calcium p-toluenesulfinate, lithium p-toluenesulfinate, 2,4,6-trimethylbenzenesulfinic acid, sodium 2,4,6-trimethylbenzenesulfinate, potassium 2,4,6-trimethylbenzenesulfinate, calcium 2,4,6-trimethylbenzenesulfinate, lithium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, calcium 2,4,6-triethylbenzenesulfinate, lithium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, calcium 2,4,6-triisopropylbenzenesulfinate, and lithium 2,4,6 -triisopropylbenzenesulfinate.

Examples of polymerization accelerators preferred for use with the chemical polymerization initiator include amines, sulfinic acid and salts thereof, copper compounds, and tin compounds.

The amines used as polymerization accelerators with the chemical polymerization initiator can be categorized into aliphatic amines, and aromatic amines having an electron withdrawing group on the aromatic ring.

Examples of the aliphatic amines include primary aliphatic amines such as n-butylamine, n-hexylamine, and n-octylamine; secondary aliphatic amines such as diisopropylamine, dibutylamine, and N-methylethanolamine: and tertiary aliphatic amines such as N-methyldiethanolamine, N-ethyldiethanolamine, N-n-butyldiethanolamine, N-lauryldiethanolamine, 2-(dimethylamino)ethylmethacrylate, N-methyldiethanolamine dimethacrylate, N-ethyldiethanolamine dimethacrylate, triethanolamine monomethacrylate, triethanolamine dimethacrylate, triethanolaminetrimethacrylate, triethanolamine, trimethylamine, triethylamine, and tributylamine. In view of ease of polymerization and storage stability, preferred are tertiary aliphatic amines, more preferably N-methyldiethanolamine and triethanolamine.

Examples of the aromatic amines having an electron withdrawing group on the aromatic ring include tertiary aromatic amines such as N,N-bis(2-hydroxyethyl)-p-toluidine, ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of the ability to impart desirable curability, the aromatic amine having an electron withdrawing group on the aromatic ring is preferably at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Examples of sulfinic acid and salts thereof used as polymerization accelerators with the chemical polymerization initiator include those exemplified above as polymerization accelerators for the photopolymerization initiator. Preferred are sodium benzenesulfinate, sodium p-toluenesulfinate, and sodium 2,4,6 -triisopropylbenzenesulfinate.

Examples of copper compounds used as polymerization accelerators with the chemical polymerization initiator include copper acetylacetonate, copper(II) acetate, copper oleate, copper(II) chloride, and copper(II) bromide.

Examples of tin compounds used as polymerization accelerators with the chemical polymerization initiator include di-n-butyltin dimaleate, di-n-octyltin dimaleate, di-n-octyltin dilaurate, and di-n-butyltin dilaurate. Preferred are di-n-octyltin dilaurate and di-n-butyltin dilaurate.

The amount of polymerization accelerator is not particularly limited. However, in view of considerations such as ease of polymerization, the polymerization accelerator is preferably 0.01 parts by mass or more, more preferably 0.1 parts by mass or more, even more preferably 1 part by mass or more, and is preferably 10 parts by mass or less, more preferably 3 parts by mass or less relative to 100 parts by mass of the polymerizable monomer.

For advantages such as enhancing the effects of the present invention, the content of the polymer in a dental mill blank of the present invention is preferably 50 volume% or more, more preferably 80 volume% or more, even more preferably 90 volume% or more of the voids in the ashed product obtained after the dental mill blank is ashed at 600°C for 2 hours. The polymer content may be 95 volume% or more, or essentially 100 volume%.

Other Components

A dental mill blank of the present invention may contain other components, such as a pH adjuster, an ultraviolet absorber, an antioxidant, a colorant, a pigment, an antimicrobial agent, an X-ray contrast agent, a thickener, and a fluorescent agent, in addition to the inorganic filler and the polymer, depending on intended use.

The pigment may be selected from known pigments used for dental composite resins or dental cements, and these may be used without any restriction. The pigment may be an inorganic pigment and/or an organic pigment. Examples of the inorganic pigment include chromates such as chrome yellow, zinc yellow, and barium yellow; ferrocyanides such as iron blue; sulfides such as silver vermilion, cadmium yellow, zinc sulfide, and cadmium red; sulfates such as barium sulfate, zinc sulfate, and strontium sulfate; oxides such as antimony white, zinc white, titanium white, red iron oxide, iron black, and chromium oxide; hydroxides such as aluminum hydroxide; silicates such as calcium silicate and ultramarine; and carbon materials such as carbon black and graphite. Examples of the organic pigment include nitrone pigments such as naphthol green B and naphthol green Y; nitro pigments such as naphthol S and lithol fast yellow 2G; insoluble azo pigments such as permanent red 4R, brilliant fast scarlet, Hansa yellow, and benzidine yellow; poorly soluble azo pigments such as lithol red, lake red C, and lake red D; soluble azo pigments such as brilliant carmine 6B, permanent red F5R, pigment scarlet 3B, and bordeaux 10B; phthalocyanine pigments such as phthalocyanine blue, phthalocyanine green, and sky blue; basic dye pigments such as rhodamine lake, malachite green lake, and methyl violet lake; and acidic dye pigments such as peacock blue lake, eosin lake, and quinoline yellow lake. The pigment may be used alone, or two or more thereof may be used in combination, and may be appropriately selected according to the desired color tone.

The content of the pigment in a dental mill blank of the present invention is appropriately adjusted according to the desired color tone, and is not particularly limited. However, the pigment content is preferably 0.000001 parts by mass or more, more preferably 0.00001 parts by mass or more, and is preferably 5 parts by mass or less, more preferably 1 part by mass or less relative to 100 parts by mass of the dental mill blank. The pigment content is preferably 0.000001 to 5 parts by mass, more preferably 0.00001 to 1 part by mass relative to 100 parts by mass of the dental mill blank.

Dental Mill Blank Producing Method

A method of production of a dental mill blank of the present invention is not particularly limited. However, for advantages such as more efficiently obtaining a dental mill blank of the desired properties, it is preferable that a dental mill blank of the present invention be produced by a method that comprises the step of contacting an inorganic filler molded product and a polymerizable monomer-containing composition with each other after preparing the inorganic filler molded product by pressing an inorganic filler, and polymerizing and curing the polymerizable monomer after making the contact.

Pressing

The inorganic filler molded product is prepared by pressing an inorganic filler. The method of pressing an inorganic filler is not particularly limited, and a known method may be used. When the inorganic filler is a combination of the inorganic filler (A) and the inorganic filler (B), a mixture of these fillers may be subjected to pressing. Specific examples of pressing methods include a method that charges an inorganic filler into a pressure mold (die) of a desired size, and uniaxially applies pressure using an upper punch and a lower punch.

The pressure of uniaxial pressing may be appropriately optimized according to conditions such as the desired size of the inorganic filler molded product to be obtained, and the type and particle size of inorganic filler. Typically, the pressure may be 10 MPa or more. Higher pressures are preferred because an increased pressure makes it easier to obtain the desired dental mill blank, and improves the stability of the inorganic filler molded product of when contacting the inorganic filler molded product and the polymerizable monomer-containing composition with each other. However, the pressure should be typically 200 MPa or less considering factors such as the size of inorganic filler molded product, productivity due to factors such as the equipment, and reduction of cracks and chipping in the inorganic filler molded product caused by, for example, friction against the mold under an excessive load. In view of this, the pressure is preferably 20 MPa or more, more preferably 25 MPa or more, and is preferably 180 MPa or less, more preferably 150 MPa or less. The pressing time may be appropriately set according to the pressure, and may be typically 1 to 120 minutes.

The pressing may be achieved by employing cold isostatic pressing (CIP). In this case, CIP may be employed by itself or with a method other than CIP, such as with the uniaxial pressing described above. Specifically, the pressing may be achieved by CIP without the uniaxial pressing, or by performing CIP after the uniaxial pressing. As a rule, pressing by CIP enables application of higher pressure than by uniaxial pressing, and the pressure can be evenly exerted three-dimensionally on the inorganic filler molded product. By employing CIP, it is therefore possible to solve the undesirable microscopic voids inside the inorganic filler molded product, and the uneven density of inorganic filler particles. The inorganic filler pressed by CIP can also have improved compression density, and the dental mill blank produced can have a high inorganic filler content. When performing CIP for pressing without uniaxial pressing, the inorganic filler may be charged into an container made of a highly elastic material such as silicone rubber or polyisoprene rubber, and may be subjected to CIP either directly or in a reduced pressure state (including a vacuum state). Likewise, in the case where uniaxial pressing is followed by CIP, the molded product from uniaxial pressing may be subjected to CIP either directly or in a reduced pressure state (including a vacuum state).

It is preferable that CIP be performed under high pressure. CIP may be performed by using, for example, a CIP apparatus capable of applying a pressure of about 1,000 MPa (e.g., a CIP apparatus manufactured by Kobe Steel, Ltd.). Higher pressures are preferred in CIP because an increased pressure makes it easier to obtain the desired dental mill blank, and improves the stability of the inorganic filler molded product of when contacting the inorganic filler molded product and the polymerizable monomer-containing composition with each other, regardless of the presence or absence of uniaxial pressing. However, considering factors such as productivity, and reduction of cracks and chipping in the inorganic filler molded product caused by, for example, friction against the mold under an excessive load, the pressure in CIP is preferably 30 MPa or more, more preferably 50 MPa or more, even more preferably 100 MPa or more, and is preferably 300 MPa or less, more preferably 250 MPa or less, even more preferably 200 MPa or less. In CIP, pressure may be applied for a time period that may be appropriately set according to the pressure. Typically, pressure may be applied for 1 to 60 minutes.

The inorganic filler molded product resulting from the pressing of the inorganic filler may have a monolayer structure or a multilayer structure. The multilayer structure may be, for example, a structure with individually pressed layers of different inorganic fillers (for example, inorganic fillers each containing both inorganic filler (A) and inorganic filler (B) but that differ in composition or other properties), or a structure with individually pressed layers of the same inorganic filler (for example, the same inorganic filler containing both inorganic filler (A) and inorganic filler (B)). In either case, the inorganic filler molded product with such a multilayer structure can be processed into a dental mill blank having layers with different color tones, different levels of transparency, and different properties. A dental mill blank having such a multilayer structure can provide a clinically useful dental prosthesis. For example, an aesthetically superior crown having an enamel color in an upper layer and a dentin color in a lower layer can be produced by machining a dental mill blank having a first layer disposed as a layer of an inorganic filler that has been adjusted to have increased transparency upon polymerization and curing of the polymerizable monomer, and a second layer disposed as a layer of an inorganic filler that has been adjusted to impart a color of dentin upon polymerization and curing of the polymerizable monomer.

The method for preparing the inorganic filler that provides the desired color after polymerization and curing is not particularly limited. For example, a method may be used that mixes and disperses a pigment (e.g., colored particles) in an inorganic filler.

The pigment may be selected from known pigments used for dental compositions. The pigment may be an inorganic pigment or an organic pigment.

Examples of the inorganic pigment include chromates such as chrome yellow, zinc yellow, and barium yellow; ferrocyanides such as iron blue; sulfides such as silver vermilion, cadmium yellow, zinc sulfide, antimony white, and cadmium red; sulfates such as barium sulfate, zinc sulfate, and strontium sulfate; oxides such as zinc white, titanium white, red iron oxide, iron black, yellow ferrous oxide, and chromium oxide; hydroxides such as aluminum hydroxide; silicates such as calcium silicate and ultramarine; and carbon materials such as carbon black and graphite.

Examples of the organic pigment include nitroso pigments such as naphthol green B and naphthol green Y; nitro pigments such as naphthol S and lithol fast yellow 2G; insoluble azo pigments such as permanent red 4R, brilliant fast scarlet, Hansa yellow, and benzidine yellow; poorly soluble azo pigments such as lithol red, lake red C, and lake red D; soluble azo pigments such as brilliant carmine 6B, permanent red F5R, pigment scarlet 3B, and bordeaux 10B; phthalocyanine pigments such as phthalocyanine blue, phthalocyanine green, and sky blue; basic dye pigments such as rhodamine lake, malachite green lake, and methyl violet lake; and acidic dye pigments such as peacock blue lake, eosin lake, and quinoline yellow lake.

These pigments may be used alone, or two or more thereof may be used in combination, and may be appropriately selected according to the desired color tone. Preferred among the foregoing examples are titanium white (e.g., Japanese Pharmacopoeia titanium oxide white), red iron oxide, iron black, and yellow ferrous oxide, which are inorganic pigments that excel in properties such as heat resistance and lightfastness.

The pigment content may be appropriately adjusted according to the desired color tone, and is not particularly limited. It is, however, preferable that the pigment content be 0.01 ppm by mass or more, more preferably 0.1 ppm by mass or more, and 5 mass% or less, more preferably 1 mass% or less, in terms of a proportion in the layer in which the pigment is mixed.

Uniform mixing and dispersing of the pigment in the inorganic filler may be achieved by using a known method of mixing and dispersing a powder, and the method may be a dry method or a wet method. However, for advantages such as more uniformly mixing and dispersing the pigment, it is preferable to use a method that includes mixing the inorganic filler and the pigment in the presence of a solvent, and subsequently removing the solvent (for example, by distillation). The mixing may follow a known method, such as by using a disperser such as a sand mill, a bead mill, an attritor, a colloid mill, a ball mill, an ultrasonic homogenizer, a homomixer, a dissolver, or a homogenizer. The mixing conditions may be appropriately selected according to conditions such as the particle sizes and amounts of the inorganic filler and pigment used; the type and amount of the solvent added; and the type of disperser. The dispersing conditions, including dispersing time, stirrer, and rotational speed, may be appropriately selected according to, for example, the dispersibility of each component. The solvent is preferably water, and/or a solvent that is compatible with water. Examples of the solvent include alcohols (for example, ethanol, methanol, isopropanol), ethers, and ketones (for example, acetone, methyl ethyl ketone).

In order to impart a desired color after polymerization and curing, it is also possible to use a method that uses an inorganic filler that itself has a specific color, such as a color glass, aside from mixing and dispersing the pigment in the inorganic filler. Examples of the inorganic filler that itself has a specific color include commercially available porcelain powders, for example, such as the porcelain powders manufactured by VITA under the trade names VM and VM7, and the porcelain powders manufactured by Kuraray Noritake Dental Inc. under the trade names Noritake Super Porcelain AAA and Cerabien ZR. These may be optionally pulverized to adjust the particle size.

In order to provide the desired transparency after polymerization and curing, it is possible to use, for example, a method that adjusts the refractive index and particle size of the inorganic filler. As is known, the transparency of an inorganic filler-dispersed resin increases as the difference between the refractive index of the inorganic filler and the refractive index of the resin itself decreases, or as the particle size deviates from the visible light wavelength (0.4 to 0.7 $\mu$m). It is accordingly preferable to use, for example, a method in which the inorganic filler to be used as a high transparency layer is an inorganic filler having a refractive index that is as close as possible to the post-polymerization and curing refractive index of the polymerizable monomer-containing composition used for impregnation. It is also possible to use a method that appropriately selects a polymerizable monomer so as to match the refractive index with that of the inorganic filler.

As a way of ensuring desired physical properties after polymerization and curing, it is possible to use, for example, a method whereby an inorganic filler having desirable glossiness is used for a layer corresponding to the enamel layer, and an inorganic filler having desirable mechanical strength is used for an inner layer corresponding to the dentin layer. By combining inorganic fillers in this fashion, a crown prosthesis can be provided that exhibits desirable durability in the mouth, and is highly useful for clinical practice.

An inorganic filler molded product of a multilayer structure can be obtained by using the following pressing method, for example. Specifically, a first inorganic filler is charged into a uniaxial pressing mold (die) fitted with a lower punch, and is pressed after setting an upper punch on the mold. After removing the upper punch, a second inorganic filler is charged onto the molded product of first inorganic filler, and is pressed with the upper punch reattached to the mold. An inorganic filler molded product of a multilayer structure can be obtained by taking it out of the mold after repeating the foregoing procedure for a number of times that depends on the number of layers needed. The applied pressure in the pressing process may be appropriately set according to conditions such as the type and amount of the inorganic filler used, and may be the same or different for each layer. Alternatively, a first inorganic filler and a second inorganic filler may be pressed together after charging the second inorganic filler onto the first inorganic filler that has been charged into the mold and had its surface leveled without being pressed.

In obtaining an inorganic filler molded product of a multilayer structure, the inorganic fillers may be pressed at once into the molded product in the manner described above, or another inorganic filler may be pressed against a molded product that has been separately molded. Aside from these methods, an inorganic filler molded product of a multilayer structure also can be obtained by laminating and pressing molded products that have been separately molded.

The shape and size of the pressed inorganic filler molded product are not particularly limited, and may be appropriately adjusted according to the shape and size of the dental mill blank described below.

Contact Between Inorganic Filler Molded product and Polymerizable Monomer-Containing Composition

The polymerizable monomer-containing composition penetrates into spaces between inorganic filler particles upon being brought into contact with the inorganic filler molded product. This produces a composition in which the inorganic filler particles are very densely dispersed in the polymerizable monomer-containing composition. In view of this, it is preferable to use an inorganic filler molded body that is not formed into a porous body by sintering and communicating.

As a rule, a particle-dispersed composite material can produce a crown restoration material that can be desirably polished to gloss, and that can remain glossy in the mouth for prolonged time periods as the particle size of the inorganic filler dispersed in the resin becomes smaller. However, high-density packing of inorganic filler in the composite material becomes difficult to achieve, and the mechanical strength and abrasion resistance of the cured product tend to decrease as the particle size of inorganic filler becomes smaller. The foregoing producing method produces a dental mill blank by contacting an inorganic filler molded product and a polymerizable monomer-containing composition with each other after preparing the inorganic filler molded product by pressing an inorganic filler, and polymerizing and curing the polymerizable monomer after making the contact. In this way, the inorganic filler can be packed in high density, and the dental mill blank can produce a dental prosthesis having desirable gloss and improved strength and abrasion resistance.

The method for contacting the inorganic filler molded product and the polymerizable monomer-containing composition with each other is not particularly limited, and this may be achieved by a method that allows the polymerizable monomer-containing composition to penetrate into spaces between inorganic filler particles. Preferred for advantages such as convenience is a method that immerses the inorganic filler molded product in the polymerizable monomer-containing composition. In this way, the polymerizable monomer-containing composition is able to gradually permeate into the inorganic filler molded product by capillary action. Here, it is preferable that the inorganic filler molded product be immersed in a reduced pressure atmosphere environment because it promotes permeation of the polymerizable monomer-containing liquid composition. By switching pressure between reduced pressure and ordinary pressure in a repeated cycle (a reduced pressure/ordinary pressure cycle), it is possible to promote further penetration of the polymerizable monomer-containing composition, and reduce the time for the polymerizable monomer-containing composition to fully permeate the inorganic filler molded product. The pressure in the reduced pressure atmosphere may be appropriately adjusted according to conditions such as the viscosity of the polymerizable monomer-containing composition, and the particle size of the inorganic filler. However, the pressure is preferably 10 kPa or less, more preferably 5 kPa or less, even more preferably 2 kPa or less, and is preferably 0.1 Pa or more, more preferably 1 Pa or more, even more preferably 10 Pa or more. The reduced pressure atmosphere may be a vacuum (for example, about $1 \times 10^{-8}$ to $1 \times 10^{-1}$ Pa).

Aside from immersing the inorganic filler molded product in the polymerizable monomer-containing composition, the polymerizable monomer-containing composition may be fed to the inorganic filler molded product in a mold under applied pressure upon pressing the inorganic filler in the mold. In this way, the polymerization and curing of the polymerizable monomer can be directly carried out in the mold following this procedure. The pressure applied to feed the polymerizable monomer-containing composition to the inorganic filler molded product is preferably 2 MPa or more, more preferably 10 MPa or more, even more preferably 20 MPa or more.

An inorganic filler molded product that has been apparently impregnated with the polymerizable monomer-containing composition after being brought into contact with the polymerizable monomer-containing composition such as by immersion may be placed under increased pressure conditions for a certain time period so that the polymerizable monomer-containing composition can more efficiently permeate into the inorganic filler molded product without leaving spaces in the inorganic filler molded product. The increased pressure can be created by using, for example, a CIP apparatus. The pressure is preferably 20 MPa or more, more preferably 50 MPa or more, even more preferably 100 MPa or more. Pressure may be repeatedly switched between increased pressure and ordinary pressure in a cycle (an increased pressure/ordinary pressure cycle).

For advantages such as more efficient permeation of the polymerizable monomer-containing composition into the inorganic filler molded product, the inorganic filler molded product and the polymerizable monomer-containing composition are contacted with each other at a temperature of preferably 0°C or more, more preferably 10°C or more, even more preferably 20°C or more. The temperature may be 30°C or more, 40°C or more, or 50°C or more. The temperature is preferably 70°C or less, more preferably 60°C or less.

The contact time of the inorganic filler molded product and the polymerizable monomer-containing composition depends on factors such as the type of inorganic filler, the size of inorganic filler molded product, the extent of permeation of the polymerizable monomer, and the contact method, and may be appropriately adjusted. For example, in the case where the inorganic filler molded product is immersed in the polymerizable monomer-containing composition, the contact time may be typically 0.1 to 240 hours. The contact time may be 0.5 to 120 hours when the contact is made in a reduced pressure atmosphere. The contact time may be 0.2 to 48 hours when the polymerizable monomer-containing composition is fed to the inorganic filler molded product under an applied pressure to the inorganic filler molded product.

Polymerizable Monomer-Containing Composition

The content of the polymerizable monomer in the polymerizable monomer-containing composition used in the producing method above may be, for example, 50 mass% or more, and is preferably 80 mass% or more, more preferably 90 mass% or more, even more preferably 95 mass% or more, particularly preferably 98 mass% or more.

Preferably, the polymerizable monomer-containing composition additionally contains the polymerization initiator, and more preferably contains the polymerization initiator and the polymerization accelerator. The polymerization initiator and the polymerization accelerator in the polymerizable monomer-containing composition may be used in the contents specified above for the polymerization initiator and the polymerization accelerator. When producing a dental mill blank containing these other components, the additional components may be incorporated in the polymerizable monomer-containing composition.

The method for preparing the polymerizable monomer-containing composition is not particularly limited. For example, the polymerizable monomer-containing composition may be prepared by mixing the polymerization initiator, the polymerization accelerator, and any other components into a polymerizable monomer.

As a rule, the polymerizable monomer-containing composition permeates into the inorganic filler molded product at a rate that increases with decrease of viscosity. Preferably, the viscosity (25°C) of the polymerizable monomer-containing composition is 10 Pa·s or less, more preferably 5 Pa·s or less, even more preferably 2 Pa·s or less. The viscosity may be adjusted by, for example, appropriately selecting a polymerizable monomer. Aside from the purpose to adjust the viscosity of the polymerizable monomer-containing composition, it is preferable that the polymerizable monomer contained in the polymerizable monomer-containing composition be selected by also taking into account the mechanical strength and the refractive index of the dental mill blank to be produced. The viscosity of the polymerizable monomer-containing composition also can be lowered by, for example, containing a solvent. In this case, the solvent may be removed in the subsequent process of reducing pressure. The permeation rate can be increased by increasing temperature and lowering the viscosity of the polymerizable monomer-containing composition. The increased temperature range may be the same temperature range specified above for the contact temperature of the inorganic filler molded product and the polymerizable monomer-containing composition.

Polymerization and Curing of Polymerizable Monomer

The desired dental mill blank can be obtained by polymerizing and curing the polymerizable monomer contained in the polymerizable monomer-containing composition having penetrated into the inorganic filler molded product as a result of being brought into contact with the inorganic filler molded product (a state where the inorganic filler molded product is impregnated with the polymerizable monomer-containing composition).

The method of polymerization and curing is not particularly limited, and may be appropriately selected from polymerization methods such as thermal polymerization, photo-polymerization, and chemical polymerization, according to factors such as the type of the polymerization initiator used. In the case of thermal polymerization, the heating temperature is not particularly limited, and may be, for example, 40 to 150°C. The heating time is not particularly limited either, and may be, for example, 1 to 70 hours. The thermal polymerization may be carried out in one step or in multiple steps. In the case of the latter, the heating temperature may be appropriately varied. In the case of photo-polymerization, the light used is not limited to a particular type of light, and may be visible light, ultraviolet light, or any other light. The photo-polymerization time is not particularly limited either, and may be, for example, 1 to 20 minutes. In view of increasing the polymerization conversion rate to obtain a dental mill blank having even higher mechanical strength, photo-polymerization may be followed by thermal polymerization.

The polymerization conversion rate can improve, and the dental mill blank produced can have even higher mechanical strength when the inorganic filler molded product impregnated with the polymerizable monomer-containing composition is subjected to polymerization and curing in an inert gas atmosphere such as a nitrogen gas atmosphere, or under reduced pressure (including vacuum conditions). In the case where polymerization and curing is carried out under reduced pressure (including vacuum conditions), it is preferable in view of advantages such as productivity that the inorganic filler molded product impregnated with the polymerizable monomer-containing composition be subjected to polymerization and curing by being packed in a vacuum pack or the like. In this case, polymerization and curing may employ thermal polymerization under applied pressure, using, for example, an autoclave.

For advantages such as further improving the mechanical strength of the dental mill blank produced, the inorganic filler molded product impregnated with the polymerizable monomer-containing composition may be subjected to polymerization and curing under applied pressure. By being placed under applied pressure, the inorganic filler molded product impregnated with the polymerizable monomer-containing composition enables the polymerizable monomer-containing composition to more effectively enter the fine spaces in the inorganic filler molded product, and the number of remaining fine bubbles can be reduced.

In the case where polymerization and curing is carried out under applied pressure, the pressure is preferably 20 MPa or more, more preferably 50 MPa or more, even more preferably 100 MPa or more, particularly preferably 200 MPa or more. It is preferable to apply as high a pressure as possible, and the pressure may be appropriately set taking into account, for example, the capacity of the pressure device actually used. Examples of the pressure device include an autoclave, a CIP apparatus, and a HIP (hot isostatic pressing) apparatus. For example, a CIP apparatus capable of applying a pressure of about 1,000 MPa (e.g., a CIP apparatus manufactured by Kobe Steel, Ltd.) may be used. When carried out under applied pressure, the polymerization and curing may employ thermal polymerization, which applies heat for polymerization and curing, or may employ photo-polymerization or chemical polymerization.

As a specific preferred example, the polymerization under applied pressure may be carried out by placing and sealing the inorganic filler molded product impregnated with the polymerizable monomer-containing composition in a vacuum pack, and polymerizing the monomer under applied pressure, using, for example, a CIP apparatus. In this way, the dental mill blank produced can have even higher mechanical strength. In the case where polymerization is carried out under applied pressure using a CIP apparatus, the processing chamber of the CIP apparatus may be heated after applying a predetermined pressure. Specifically, for example, a predetermined pressure is applied at room temperature to the inorganic filler molded product impregnated with the polymerizable monomer-containing composition, inside the processing chamber of a CIP apparatus, and the chamber is heated over a time period of from about 30 minutes to about 24 hours until it reaches a predetermined target temperature. The target temperature may be, for example, 80 to 180°C. The polymerization time and the target temperature may be set taking into account factors such as the decomposition temperature of the polymerization initiator used.

The cured product obtained after the foregoing polymerization and curing process may be used directly as a dental mill blank. However, by performing a heat treatment after polymerization and curing, the stress-induced strain that generates in the cured product can be relaxed, and damage that may occur while machining the dental mill blank into a dental prosthesis or during clinical use of the dental prosthesis can be reduced. The heat treatment temperature may be, for example, 80 to 150°C. The heat treatment may be performed for, for example, 10 to 120 minutes.

The cured product from the foregoing polymerization and curing process may be prepared into a dental mill blank by being optionally cut and milled into the desired size, and polishing the surface.

Dental Mill Blank

A dental mill blank of the present invention has use in dentistry, and may be used for, for example, fabrication of dental prostheses through processes such as cutting, carving, and milling. With the dental mill blank, a dental prosthesis can be provided that excels in mechanical strength and aesthetic quality. Examples of such dental prostheses include crown restorations such as inlays, onlays, veneers, crowns, and bridges; abutment teeth, dental posts, dentures, denture bases, and implant parts (e.g., fixtures, abutments). Preferably, the dental mill blank is processed with a dental CAD/CAM system. Examples of the dental CAD/CAM system include the CEREC system manufactured by Sirona Dental Systems Inc., and the Katana system manufactured by Kuraray Noritake Dental Inc.

The shape of a dental mill blank of the present invention is not particularly limited, and may be appropriately set according to considerations such as intended use. For example, a dental mill blank of the present invention may have a prism shape such as a triangular prism, a quadrangular prism, or a hexagonal prism; or a cylindrical shape such as a disc (disc-like) shape.

The size of a dental mill blank of the present invention is not particularly limited, and may be, for example, a size that can be set in a commercially available dental CAD/CAM system. Specific examples of the size of a dental mill blank of the present invention include a 40 mm × 20 mm × 15 mm prism suited for fabrication of, for example, single bridges, a 17 mm × 10 mm × 10 mm prism suited for fabrication of, for example, inlays and onlays, a 14 mm × 18 mm × 20 mm prism suited for fabrication of, for example, full crowns, and a disc having a diameter of 100 mm and a thickness of 10 to 28 mm suited for fabrication of, for example, long-span bridges and denture bases.

EXAMPLES

The following specifically describes the present invention by way of Examples and Comparative Examples. It should be noted that the present invention is in no way limited by the following Examples. Details of the components used are as follows.

Inorganic Filler

UF2.0: Barium glass (average primary particle diameter 2.0 $\mu$m, manufactured by Schott)
UF1.5: Barium glass (average primary particle diameter 1.5 $\mu$m, manufactured by Schott)
UF1.0: Barium glass (average primary particle diameter 1.0 $\mu$m, manufactured by Schott)
SM3.5: Lanthanum glass (average primary particle diameter 3.5 $\mu$m, manufactured by Schott)
NF180: Barium glass (average primary particle diameter 0.18 $\mu$m, manufactured by Schott)
Ox-50: Fine silica particles (average primary particle diameter 0.04 $\mu$m, manufactured by Nippon Aerosil Co., Ltd.)

Surface Treatment Agent

$\gamma$-MPS: $\gamma$-Methacryloyloxypropyltrimethoxysilane (manufactured by Shin-Etsu Chemical Co., Ltd.)

Binder

UDMA: [2,2,4-Trimethylhexamethylenebis(2-carbamoyloxyethyl)] dimethacrylate

Polymerizable monomer

UDMA: [2,2,4-Trimethylhexamethylenebis(2-carbamoyloxyethyl)] dimethacrylate
3G: Triethylene glycol dimethacrylate

Polymerization initiator

THP: 1,1,3,3-Tetramethylbutylhydroperoxide (manufactured by NOF Corporation)
BPO: Benzoyl peroxide (manufactured by NOF Corporation)

Production Example 1

Production of Inorganic Filler (F1)

For production of inorganic filler (F1), 100 parts by mass of a mixture of 75 parts by volume of UF2.0 and 25 parts by volume of NF180 was dispersed in 300 parts by mass of ethanol, and the resulting mixture was stirred at room temperature for 2 hours after adding 2.5 parts by mass of γ-MPS, 0.15 parts by mass of acetic acid, and 5 parts by mass of water. The solvent was distilled away under reduced pressure, and a surface treatment was carried out by drying at 90°C for 3 hours to obtain an inorganic filler (F1).

Production Example 2

Production of Inorganic Filler (F2)

For production of inorganic filler (F2), 100 parts by mass of a mixture of 75 parts by volume of UF1.0 and 25 parts by volume of NF180 was dispersed in 300 parts by mass of ethanol, and the resulting mixture was stirred at room temperature for 2 hours after adding 5 parts by mass of γ-MPS, 0.15 parts by mass of acetic acid, and 5 parts by mass of water. The solvent was distilled away under reduced pressure, and a surface treatment was carried out by drying at 90°C for 3 hours to obtain an inorganic filler (F2).

Production Example 3

Production of Inorganic Filler (F3)

For production of inorganic filler (F3), 100 parts by mass of a mixture of 75 parts by volume of SM3.5 and 25 parts by volume of NF180 were dispersed in 300 parts by mass of ethanol, and the resulting mixture was stirred at room temperature for 2 hours after adding 1.8 parts by mass of γ-MPS, 0.15 parts by mass of acetic acid, and 5 parts by mass of water. The solvent was distilled away under reduced pressure, and a surface treatment was carried out by drying at 90°C for 3 hours to obtain an inorganic filler (F3).

Production Example 4

Production of Inorganic Filler (F4)

For production of inorganic filler (F4), 100 parts by mass of a mixture of 80 parts by volume of UF2.0 and 20 parts by volume of Ox-50 was dispersed in 300 parts by mass of ethanol, and the resulting mixture was stirred at room temperature for 2 hours after adding 2.5 parts by mass of γ-MPS, 0.15 parts by mass of acetic acid, and 5 parts by mass of water. The solvent was distilled away under reduced pressure, and a surface treatment was carried out by drying at 90°C for 3 hours to obtain an inorganic filler (F4).

Production Example 5

Production of Inorganic Filler (F5)

For production of inorganic filler (F5), 100 parts by mass of UF2.0 was dispersed in 300 parts by mass of ethanol, and the resulting mixture was stirred at room temperature for 2 hours after adding 2.5 parts by mass of γ-MPS, 0.15 parts by mass of acetic acid, and 5 parts by mass of water. The solvent was distilled away under reduced pressure, and a surface treatment was carried out by drying at 90°C for 3 hours to obtain an inorganic filler (F5).

Production Example 6

Production of Inorganic Filler (F6)

For production of inorganic filler (F6), 100 parts by mass of SM3.5 was dispersed in 300 parts by mass of ethanol, and the resulting mixture was stirred at room temperature for 2 hours after adding 1.8 parts by mass of γ-MPS, 0.15 parts by mass of acetic acid, and 5 parts by mass of water. The solvent was distilled away under reduced pressure, and a surface treatment was carried out by drying at 90°C for 3 hours to obtain an inorganic filler (F6).

Production Example 7

Production of Inorganic Filler (F7)

For production of inorganic filler (F7), 100 parts by mass of NF180 was dispersed in 300 parts by mass of ethanol, and the resulting mixture was stirred at room temperature for 2 hours after adding 2.5 parts by mass of γ-MPS, 0.15 parts by mass of acetic acid, and 5 parts by mass of water. The solvent was distilled away under reduced pressure, and a surface treatment was carried out by drying at 90°C for 3 hours to obtain an inorganic filler (F7).

Production Example 8

Production of Inorganic Filler (F8)

For production of inorganic filler (F8), 100 parts by mass of Ox-50 was dispersed in 300 parts by mass of ethanol, and the resulting mixture was stirred at room temperature for 2 hours after adding 7 parts by mass of γ-MPS, 0.15 parts by mass of acetic acid, and 5 parts by mass of water. The solvent was distilled away under reduced pressure, and a surface treatment was carried out by drying at 90°C for 3 hours to obtain an inorganic filler (F8).

Production Example 9

Production of Inorganic Filler (F9)

For production of inorganic filler (F9), 100 parts by mass of a mixture of 75 parts by volume of UF2.0 and 25 parts by volume of UF1.0 was dispersed in 300 parts by mass of ethanol, and the resulting mixture was stirred at room temperature for 2 hours after adding 2.5 parts by mass of γ-MPS, 0.15 parts by mass of acetic acid, and 5 parts by mass of water. The solvent was distilled away under reduced pressure, and a surface treatment was carried out by drying at 90°C for 3 hours to obtain an inorganic filler (F9).

Production Example 10

Production of Inorganic Filler Complex (F10)

For production of inorganic filler complex (F10), 100 parts by mass of a mixture of 75 parts by volume of UF2.0 and 25 parts by volume of NF180 was dispersed in 300 parts by mass of ethanol, and the resulting mixture was stirred at room temperature for 2 hours after adding 2.5 parts by mass of γ-MPS, 0.15 parts by mass of acetic acid, and 5 parts by mass of water. The mixture was further stirred at room temperature for 10 minutes after adding 1 part by mass of UDMA as a binder. The solvent was distilled away under reduced pressure, and the remaining product was dried at 90°C for 3 hours to obtain an inorganic filler complex (F10) having the inorganic filler surface-treated with surface treatment agent, and the binder present on the surface of the complex.

Production Example 11

Production of Inorganic Filler Complex (F11)

For production of inorganic filler complex (F11), 100 parts by mass of a mixture of 76 parts by volume of UF1.5 and 24 parts by volume of Ox-50 was dispersed in 217 parts by mass of toluene, and the resulting mixture was heated under reflux for 2 hours after adding 3.3 parts by mass of γ-MPS. The solvent was distilled away under reduced pressure, and the remaining product was dried at 90°C for 3 hours to obtain an inorganic filler complex (F11) having the inorganic filler surface-treated with the surface treatment agent.

Production Example 12

Production of Polymerizable Monomer-Containing Composition (P1)

For preparation of polymerizable monomer-containing composition (P1), 1 part by mass of THP was dissolved as polymerization initiator in 80 parts by mass of UDMA and 19 parts by mass of 3G.

Production Example 13

Production of Polymerizable Monomer-Containing Composition (P2)

For preparation of polymerizable monomer-containing composition (P2), 1 part by mass of THP was dissolved as polymerization initiator in 99 parts by mass of UDMA.

Production Example 14

Production of Polymerizable Monomer-Containing Composition (P3)

For preparation of polymerizable monomer-containing composition (P3), 1 part by mass of BPO was dissolved as polymerization initiator in 80 parts by mass of UDMA and 19 parts by mass of 3G.

Production Example 15

Production of Polymerizable Monomer-Containing Composition (P4)

For preparation of polymerizable monomer-containing composition (P4), 1.5 parts by mass of BPO was dissolved as polymerization initiator in 70 parts by mass of UDMA and 30 parts by mass of 3G.

Example 1

(1) In Example 1, 7.0 g of the inorganic filler (F1) obtained in Production Example was laid on a lower punching rod of a pressure mold having a 14.5 mm × 18 mm rectangular hole. After leveling the powder by tapping, an upper punching rod was set above, and a pressure of 10 kN (38.3 MPa) was uniaxially applied for 2 minutes using a desktop pressing machine. The resulting molded product which was packed inorganic filler (F1) was taken out of the mold by removing the upper and lower punching rods, and was pressed under a pressure of 170 MPa for 5 minutes by CIP to obtain an inorganic filler molded product.
(2) The inorganic filler molded product was immersed in polymerizable monomer-containing composition (P1). After removing air under reduced pressure (10 hPa), the whole was allowed to stand at 70°C for 48 hours to obtain an inorganic filler molded product impregnated with the polymerizable monomer-containing composition (polymerizable monomer impregnated molded product). The polymerizable monomer impregnated molded product was heated at 110°C for 7 hours, and at 150°C for 7 hours with a hot-air dryer to obtain the desired dental mill blank, rectangular in shape.

Examples 2 to 5 and Comparative Examples 1 to 6

Rectangular dental mill blanks were obtained in the same manner as in Example 1, except that the inorganic fillers and the polymerizable monomer-containing compositions shown in Table 1 were used.
In Example 5, the inorganic filler molded product in step (2) was immersed in polymerizable monomer-containing composition (P3), and, after removing air under reduced pressure (10 hPa), the whole was allowed to stand at 40°C for 48 hours to obtain a polymerizable monomer impregnated molded product. The polymerizable monomer impregnated molded product was heated at 55°C for 18 hours, and at 110°C for 3 hours with a hot-air dryer to obtain the desired dental mill blank.

Example 6

A rectangular dental mill blank was obtained in the same manner as in Example 4, except that inorganic filler complex (F10) was used instead of inorganic filler (F1).

Comparative Example 7

(1) In Comparative Example 7, 200 g of the inorganic filler complex (F11) obtained in Production Example was laid on a lower punching rod of a pressure mold having a circular hole with a diameter $\phi$ of 200 mm. After leveling the powder by tapping, an upper punching rod was set above, and a pressure of 300 kN (26.5 MPa) was uniaxially applied for 5 minutes using a desktop pressing machine. The resulting molded product which was packed inorganic filler complex (F11) was taken out of the mold by removing the upper and lower punching rods, and was pressed under a pressure of 350 MPa for 20 minutes by CIP to obtain an inorganic filler molded product.

(2) The inorganic filler molded product was immersed in polymerizable monomer-containing composition (P4). After removing air under reduced pressure (10 hPa), the whole was allowed to stand at 70°C for 48 hours to obtain an inorganic filler molded product impregnated with the polymerizable monomer-containing composition (polymerizable monomer impregnated molded product). The polymerizable monomer impregnated molded product was taken out of the mold and placed on a glass slide, and subjected to photo-polymerization by applying light for 60 minutes using a UV generator (a blacklight fluorescence lamp manufactured by Toshiba Corporation). After exposure, the polymerizable monomer impregnated molded product was heated at 70°C for 24 hours, and at 110°C for 5 hours with a hot-air dryer to obtain the desired dental mill blank.

Test Example 1

Crack Evaluation

Dental mill blanks were produced, ten each for Examples and Comparative Examples, and the presence or absence of cracks in the dental mill blanks was evaluated as follows. In one evaluation, the dental mill blanks were visually inspected for the presence or absence of surface cracks. The dental mill blanks were also evaluated for the presence or absence of internal cracks, using an X-ray imager. This was followed by counting of dental mill blanks that had a surface crack or an internal crack, or both. The results are presented in Tables 1 to 3.

Test Example 2

Flexural Strength Measurement (without immersion or after immersion in water at 37°C (1 month))

The flexural strength of each dental mill blank was measured as follows. Specifically, the dental mill blank was fabricated into a specimen (1.2 mm × 4 mm × 14 mm) using a diamond cutter, and polished with a #2000 abrasive paper. The specimen was set in an multi-purpose tester (Shimadzu Corporation), either directly (without immersion) or after being immersed in 37°C water for 1 month (after immersion in water at 37°C (1 month)). The specimen was then measured for flexural strength by a three-point flexural test conducted at a crosshead speed of 1 mm/min and a span length of 12 mm (the JDMAS 245:2017 specifications were applied). The results are presented in Tables 1 to 3. The flexural strength is preferably 250 MPa or more, more preferably 270 MPa or more, even more preferably 280 MPa or more, particularly preferably 290 MPa or more for the non-immersed specimens. For specimens immersed in water at 37°C for 1 month, the flexural strength is preferably 200 MPa or more, more preferably 220 MPa or more, even more preferably 240 MPa or more.

Test Example 3

Measurement of Gloss Retention (Glossiness)

The gloss retention of each dental mill blank was measured as follows. Specifically, the dental mill blank was fabricated into a specimen (10 mm × 10 mm × 2 mm) using a diamond cutter. A clean, smooth surface of the specimen was polished under dry conditions, using a #1000 abrasive paper, a #2000 abrasive paper, and a #3000 abrasive paper, in this order. The surface was finished by polishing with a lapping film. The glossiness of the specimen was measured before and after a toothbrush abrasion test (toothbrush: Between Lion, regular hardness, manufactured by Lion Corporation; toothpaste: Dentor Clear MAX manufactured by Lion Corporation; load: 250 g; test solution: distilled water/toothpaste = 90/10 (volume/volume, 50 mL); brushing cycle: 40,000 cycles), using a glossmeter (VG-2000, manufactured by Nippon Denshoku Industries Co., Ltd.). Here, 60-degree specular gloss (Gs(60°)) was taken as a measure of glossiness. The percentage gloss retention (%) was calculated by dividing the glossiness after the toothbrush abrasion test by the glossiness before the test. The results are presented in Tables 1 to 3. The percentage gloss retention is preferably 70% or more, more preferably 80% or more, even more preferably 90% or more, particularly preferably 93% or more.

[Table 1]

| Inorganic filler | | | | Example | | | | | Comparative Example | | | | | |
|---|---|---|---|---|---|---|---|---|---|---|---|---|---|---|---|
| | | | | 1 | 2 | 3 | 4 | 5 | 1 | 2 | 3 | 4 | 5 | 6 |
| | | | Type | (F1) | (F2) | (F3) | (F1) | (F1) | (F4) | (F5) | (F6) | (F7) | (F8) | (F9) |
| | UF2.0 | (Average primary particle diameter: 2.0 $\mu$m) | Volume% *1) | 75 | | | 75 | 75 | 80 | 100 | | | | 75 |
| | UF1.0 | (Average primary particle diameter: 1.0 $\mu$m) | Volume% *1) | | 75 | | | | | | | | | 25 |
| | SM3.5 | (Average primary particle diameter: 3.5 $\mu$m) | Volume% *1 | | | 75 | | | | | 100 | | | |
| | NF180 | (Average primary particle diameter: 0.18 $\mu$m) | Volume% *1) | 25 | 25 | 25 | 25 | 25 | | | | 100 | | |
| | Ox-50 | (Average primary particle diameter: 0.04 $\mu$m) | Volume% *1) | | | | | | 20 | | | | 100 | |
| Polymerizable monomer-containing composition | | | | | | | | | | | | | | |
| | | | Type | (P1) | (P1) | (P1) | (P2) | (P3) | (P1) | (P1) | (P1) | (P1) | (P3) | (P1) |

EP 3 689 291 B1

| Dental mill blank | | | Example | | | | | Comparative Example | | | | | |
|---|---|---|---|---|---|---|---|---|---|---|---|---|---|
| | | | 1 | 2 | 3 | 4 | 5 | 1 | 2 | 3 | 4 | 5 | 6 |
| | Porosity after ashing *2) | Volume% | 33 | 34 | 31 | 33 | 33 | 23 | 42 | 40 | 51 | 48 | 41 |
| | Crack evaluation | Number of cracks | 0 | 0 | 0 | 0 | 0 | 1 | 5 | 9 | 3 | 3 | 4 |
| | Flexural strength (without immersion) | MPa | 284 | 272 | 293 | 291 | 280 | 223 | 238 | 234 | 216 | 228 | 234 |
| | Flexural strength (after immersion in 37°C water (1 month)) | MPa | 242 | 235 | 254 | 248 | 239 | 188 | 194 | 201 | 158 | 168 | 191 |
| | Gloss retention (Glossiness) (before toothbrush abrasion test) | % | 97 | 98 | 94 | 98 | 97 | 79 | 78 | 73 | 84 | 92 | 78 |
| | Gloss retention (Glossiness) (after toothbrush abrasion test) | % | 92 | 92 | 88 | 94 | 93 | 70 | 60 | 49 | 71 | 87 | 60 |
| | Percentage gloss retention | % | 95 | 94 | 94 | 96 | 96 | 89 | 77 | 67 | 85 | 95 | 77 |

*1) Volume fraction in Inorganic filler
*2) Porosity after 2-hour ashing at 600°C

EP 3 689 291 B1

23

[Table 2]

| | | | | Example |
|---|---|---|---|---|
| | | | | 6 |
| Inorganic filler complex | | | Type | (F10) |
| Inorganic filler (before surface treatment) | | | Parts by mass | 100 |
| | UF2.0 | (Average primary particle diameter: 2.0 μm) | Volume% *1) | 75 |
| | NF180 | (Average primary particle diameter: 0.18 μm) | Volume% *1) | 25 |
| Surface treatment agent | γ-MPS | | Parts by mass | 2.5 |
| Binder | UDMA | | Parts by mass | 1 |
| Polymerizable monomer-containing composition | | | Type | (P2) |
| Dental mill blank | | | | |
| | | Porosity after ashing *2) | Volume% | 30 |
| | | Crack evaluation | Number of cracks | 0 |
| | | Flexural strength (without immersion) | MPa | 342 |
| | | Flexural strength (after immersion in 37°C water (1 month)) | MPa | 286 |
| | | Gloss retention (Glossiness) (before toothbrush abrasion test) | % | 98 |
| | | Gloss retention (Glossiness) (after toothbrush abrasion test) | % | 95 |
| | | Percentage gloss retention | % | 97 |

*1) Volume fraction in Inorganic filler
*2) Porosity after 2-hour ashing at 600°C

[Table 3]

| | | | | Comparative Example |
|---|---|---|---|---|
| | | | | 7 |
| Inorganic filler complex | | | Type | (F11) |
| Inorganic filler (before surface treatment) | | | Parts by mass | 100 |
| | UF1.5 | (Average primary particle diameter: 1.5 μm) | Volume% *1) | 76 |
| | Ox-50 | (Average primary particle diameter: 0.04 μm) | Volume% *1) | 24 |
| Surface treatment agent | | | | |
| | γ-MPS | | Parts by mass | 3.3 |
| Polymerizable monomer-containing composition | | | Type | (P4) |

(continued)

| | | | Comparative Example |
|---|---|---|---|
| | | | 7 |
| Dental mill blank | | | |
| | Porosity after ashing *2) | Volume% | 20 |
| | Crack evaluation | Number of cracks | 2 |
| | Flexural strength (without immersion) | MPa | 209 |
| | Flexural strength (after immersion in 37°C water (1 month)) | MPa | 173 |
| | Gloss retention (Glossiness) (before toothbrush abrasion test) | % | 72 |
| | Gloss retention (Glossiness) (after toothbrush abrasion test) | % | 61 |
| | Percentage gloss retention | % | 85 |
| *1) Volume fraction in Inorganic filler | | | |
| *2) Porosity after 2-hour ashing at 600°C | | | |

The dental mill blanks of Examples 1 to 6 had no observable surface and internal cracks. The dental mill blanks of Examples 1 to 6 also had high flexural strength before and after immersion in 37°C water for 1 month, and the mechanical strength was desirable. The gloss retention was also desirable as demonstrated by the high glossiness observed before and after the toothbrush abrasion test. The dental mill blanks of Examples 1 to 6 also had high percentages of gloss retention before and after the test, and the aesthetic quality was desirable.

In contrast, the dental mill blanks of Comparative Examples 1 to 6 were inferior to the dental mill blanks of Examples in terms of a number of properties, including cracking, mechanical strength, and aesthetic quality. With a porosity of less than 25 volume%, the inorganic filler particles pack themselves too densely, and make the dental mill blank hard and brittle, with the result that the flexural strength decreases, as observed in the dental mill blank of Comparative Example 7. A dental mill blank with a porosity of less than 25 volume% is hard and brittle, and suffers from poor gloss retention because of increased occurrence of surface roughness after toothbrush abrasion, as observed in the dental mill blank of Comparative Example 7. When the molded product is overpressurized for its fabrication to make the porosity less than 25 volume%, the cracking rate increases as a result of the excessive load exerted on the molded product.

**Claims**

1. A dental mill blank comprising an inorganic filler and a polymer, **characterised in that**: the dental mill blank has a porosity of 25 to 35 volume% after 2-hour ashing at 600°C; and that the inorganic filler comprises an inorganic filler (A) and an inorganic filler (B), wherein the dental mill blank satisfies the following formulae (I) and (II),

$$0.12 \leq a \leq 0.70 \qquad (I)$$

$$3 \leq b/a \qquad (II),$$

where a is an average primary particle diameter of the inorganic filler (A) in micrometers, and b is an average primary particle diameter of the inorganic filler (B) in micrometers.

2. The dental mill blank according to claim 1, wherein the inorganic filler (A) and the inorganic filler (B) have a volume-to-volume content ratio (A)/(B) of 5/95 to 50/50.

3. The dental mill blank according to claim 1 or 2, wherein the dental mill blank satisfies the following formula (III),

$$0.8 \leq b \leq 10 \qquad \text{(III)}.$$

4. The dental mill blank according to any one of claims 1 to 3, wherein the polymer is formed of at least one selected from the group consisting of a (meth)acrylate polymerizable monomer and a (meth)acrylamide polymerizable monomer.

5. The dental mill blank according to any one of claims 1 to 4, wherein the inorganic filler is forming an inorganic filler complex having a binder on its surface.

6. The dental mill blank according to claim 5, wherein the binder is at least one selected from the group consisting of a polymerizable monomer, a polymer of a polymerizable monomer, a wax, and a plasticizer.

7. The dental mill blank according to claim 5, wherein the binder is a polymer of at least one of polymerizable monomers forming the polymer contained in the dental mill blank.

8. A method for producing the dental mill blank of any one of claims 1 to 7, comprising the step of contacting an inorganic filler molded product and a polymerizable monomer-containing composition with each other after preparing the inorganic filler molded product by pressing an inorganic filler, and polymerizing and curing the polymerizable monomer after making the contact, **characterised in that**: the inorganic filler comprises an inorganic filler (A) and an inorganic filler (B), wherein the inorganic filler (A) and inorganic filler (B) satisfy the following formulae (I) and (II),

$$0.12 \leq a \leq 0.70 \qquad \text{(I)}$$

$$3 \leq b/a \qquad \text{(II)},$$

where a is the average primary particle diameter of the inorganic filler (A) in micrometers, and b is the average primary particle diameter of the inorganic filler (B) in micrometers.

9. The method according to claim 8, wherein the pressing comprises cold isostatic pressing (CIP).

10. The method according to claim 8 or 9, wherein the inorganic filler subjected to the pressing is forming an inorganic filler complex having a binder on its surface.

11. The method according to claim 10, wherein the binder on the inorganic filler complex formed by the inorganic filler subjected to the pressing is at least one selected from the group consisting of a polymerizable monomer, a polymer of a polymerizable monomer, a wax, and a plasticizer.

12. The method according to claim 10, wherein the binder on the inorganic filler complex formed by the inorganic filler subjected to the pressing is at least one of polymerizable monomers forming the polymer contained in the dental mill blank.

**Patentansprüche**

1. Dentalfräsrohling, umfassend einen anorganischen Füllstoff und ein Polymer, **dadurch gekennzeichnet, dass**: der Dentalfräsrohling eine Porosität von 25 bis 35 Vol.-% nach 2 Stunden Veraschen bei 600 °C aufweist; und dass der anorganische Füllstoff einen anorganischen Füllstoff (A) und einen anorganischen Füllstoff (B) umfasst, wobei der Dentalfräsrohling den nachstehenden Formeln (I) und (II) entspricht,

$$0{,}12 \leq a \leq 0{,}70 \qquad \text{(I)}$$

$$3 \leq b/a \qquad (II),$$

wobei a ein mittlerer Primärpartikeldurchmesser des anorganischen Füllstoffs (A) in Mikrometer ist und b ein mittlerer Primärpartikeldurchmesser des anorganischen Füllstoffs (B) in Mikrometer ist.

2. Dentalfräsrohling gemäß Anspruch 1, wobei der anorganische Füllstoff (A) und der anorganische Füllstoff (B) ein Volumen-zu-Volumen-Gehaltsverhältnis (A)/(B) von 5/95 bis 50/50 aufweisen.

3. Dentalfräsrohling gemäß Anspruch 1 oder 2, wobei der Dentalfräsrohling der nachstehenden Formel (III) entspricht,

$$0,8 \leq b \leq 10 \qquad (III).$$

4. Dentalfräsrohling gemäß einem der Ansprüche 1 bis 3, wobei das Polymer aus wenigstens einem ausgewählt aus der Gruppe bestehend aus einem polymerisierbaren (Meth)acrylatmonomer und einem polymerisierbaren (Meth)acrylamidmonomer gebildet ist.

5. Dentalfräsrohling gemäß einem der Ansprüche 1 bis 4, wobei der anorganische Füllstoff einen Anorganischer-Füllstoff-Komplex mit einem Bindemittel an seiner Oberfläche bildet.

6. Dentalfräsrohling gemäß Anspruch 5, wobei das Bindemittel wenigstens eines ausgewählt aus der Gruppe bestehend aus einem polymerisierbaren Monomer, einem Polymer eines polymerisierbaren Monomers, einem Wachs und einem Weichmacher ist.

7. Dentalfräsrohling gemäß Anspruch 5, wobei das Bindemittel ein Polymer von wenigstens einem von polymerisierbaren Monomeren, die das in dem Dentalfräsrohling enthaltenen Polymer bilden, ist.

8. Verfahren zur Herstellung des Dentalfräsrohlings gemäß einem der Ansprüche 1 bis 7, umfassend den Schritt des Inkontaktbringens eines Anorganischer-Füllstoff-Formkörpers und einer polymerisierbares-Monomerenthaltenden Zusammensetzung miteinander nach Herstellen des Anorganischer-Füllstoff-Formkörpers durch Pressen eines anorganischen Füllstoffs, und Polymerisieren und Härten des polymerisierbaren Monomers nach Bilden des Kontakts, **dadurch gekennzeichnet, dass**: der anorganische Füllstoff einen anorganischen Füllstoff (A) und einen anorganischen Füllstoff (B) umfasst, wobei der anorganische Füllstoff (A) und der anorganische Füllstoff (B) den nachstehenden Formeln (I) und (II) entsprechen,

$$0,12 \leq a \leq 0,70 \qquad (I)$$

$$3 \leq b/a \qquad (II),$$

wobei a der mittlere Primärpartikeldurchmesser des anorganischen Füllstoffs (A) in Mikrometer ist und b der mittlere Primärpartikeldurchmesser des anorganischen Füllstoffs (B) in Mikrometer ist.

9. Verfahren gemäß Anspruch 8, wobei das Pressen kaltisostatisches Pressen (CIP) umfasst.

10. Verfahren gemäß Anspruch 8 oder 9, wobei der dem Pressen unterzogene anorganische Füllstoff einen Anorganischer-Füllstoff-Komplex mit einem Bindemittel an seiner Oberfläche bildet.

11. Verfahren gemäß Anspruch 10, wobei das Bindemittel an dem Anorganischer-Füllstoff-Komplex, der von dem Pressen unterzogenen anorganischen Füllstoff gebildet wird, wenigstens eines ausgewählt aus der Gruppe bestehend aus einem polymerisierbaren Monomer, einem Polymer eines polymerisierbaren Monomers, einem Wachs und einem Weichmacher ist.

12. Verfahren gemäß Anspruch 10, wobei das Bindemittel an dem Anorganischer-Füllstoff-Komplex, der von dem Pressen unterzogenen anorganischen Füllstoff gebildet wird, wenigstens eines von polymerisierbaren Monomeren, die das in dem Dentalfräsrohling enthaltenen Polymer bilden, ist.

**Revendications**

1. Ébauche dentaire comprenant une charge inorganique et un polymère, **caractérisée en ce que** : l'ébauche dentaire possède une porosité de 25 à 35 % en volume après 2 heures de calcination à 600 °C ; et **en ce que** la charge inorganique comprend une charge inorganique (A) et une charge inorganique (B), l'ébauche dentaire satisfaisant les formules suivantes (I) et (II),

$$0,12 \leq a \leq 0,70 \qquad (I)$$

$$3 \leq b/a \qquad (II),$$

où a est un diamètre moyen de particule primaire de la charge inorganique (A) en micromètres, et b est un diamètre moyen de particule primaire de la charge inorganique (B) en micromètres.

2. Ébauche dentaire selon la revendication 1, la charge inorganique (A) et la charge inorganique (B) possédant un rapport de teneur volume sur volume (A)/(B) de 5/95 à 50/50.

3. Ébauche dentaire selon la revendication 1 ou 2, l'ébauche dentaire satisfaisant la formule suivante (III)

$$0,8 \leq b \leq 10 \qquad (III).$$

4. Ébauche dentaire selon l'une quelconque des revendications 1 à 3, le polymère étant formé d'au moins l'un choisi dans le groupe constitué par un monomère polymérisable de type (méth)acrylate et un monomère polymérisable de type (méth)acrylamide.

5. Ébauche dentaire selon l'une quelconque des revendications 1 à 4, la charge inorganique formant un complexe de charge inorganique possédant un liant sur sa surface.

6. Ébauche dentaire selon la revendication 5, le liant étant au moins l'un choisi dans le groupe constitué par un monomère polymérisable, un polymère d'un monomère polymérisable, une cire et un plastifiant.

7. Ébauche dentaire selon la revendication 5, le liant étant un polymère d'au moins l'un de monomères polymérisables formant le polymère contenu dans l'ébauche dentaire.

8. Procédé pour la production de l'ébauche dentaire selon l'une quelconque des revendications 1 à 7, comprenant l'étape de mise en contact d'un produit moulé de charge inorganique et d'une composition contenant un monomère polymérisable l'un avec l'autre après la préparation du produit moulé de charge inorganique par pressage d'une charge inorganique, et polymérisation et durcissement du monomère polymérisables après la mise en contact, **caractérisé en ce que** : la charge inorganique comprend une charge inorganique (A) et une charge inorganique (B), la charge inorganique (A) et la charge inorganique (B)satisfaisant les formules suivantes (I) et (II),

$$0,12 \leq a \leq 0,70 \qquad (I)$$

$$3 \leq b/a \qquad (II),$$

où a est le diamètre moyen de particule primaire de la charge inorganique (A) en micromètres, et b est le diamètre moyen de particule primaire de la charge inorganique (B) en micromètres.

9. Procédé selon la revendication 8, le pressage comprenant un pressage isostatique à froid (CIP).

10. Procédé selon la revendication 8 ou 9, la charge inorganique soumise au pressage formant un complexe de charge inorganique possédant un liant sur sa surface.

**11.** Procédé selon la revendication 10, le liant sur le complexe de charge inorganique formé par la charge inorganique soumise au pressage étant au moins l'un choisi dans le groupe constitué par un monomère polymérisable, un polymère d'un monomère polymérisable, une cire et un plastifiant.

**12.** Procédé selon la revendication 10, le liant sur le complexe de charge inorganique formé par la charge inorganique soumise au pressage étant au moins l'un de monomères polymérisables formant le polymère contenu dans l'ébauche dentaire.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017154850 A
- WO 2014021343 A1

- WO 2012042911 A